(19) **Europäisches Patentamt**

**European Patent Office**

**Office européen des brevets**

(11) **EP 2 392 927 A1**

(12) **EUROPEAN PATENT APPLICATION**

(43) Date of publication:
**07.12.2011 Bulletin 2011/49**

(51) Int Cl.:
***G01N 33/92*** *(2006.01)*

(21) Application number: **11157846.4**

(22) Date of filing: **11.03.2011**

<table>
<tr>
<td>

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
• **F. Hoffmann-La Roche AG**
**4070 Basel (CH)**
Designated Contracting States:
**AL AT BE BG CH CY CZ DK EE ES FI FR GB GR HR HU IE IS IT LI LT LU LV MC MK MT NL NO PL PT RO RS SE SI SK SM TR**
• **Roche Diagnostics GmbH**
**68305 Mannheim (DE)**
Designated Contracting States:
**DE**

</td>
<td>

(72) Inventor: **The designation of the inventor has not yet been filed**

(74) Representative: **Huhn, Michael et al**
**Patentanwalt**
**N4, 1**
**68161 Mannheim (DE)**

</td>
</tr>
</table>

(54) **Diagnosis of metabolic disorders in HIV infected patients**

(57) The present invention relates to a method of diagnosing metabolic alterations potentially leading to a cardiac complication or death in a human immunodeficiency virus (HIV) infected subject. Preferably, the HIV infected subject is under an antiretroviral therapy, in general an active antiviral therapy including administration of combinations of antiretroviral drugs for HIV therapy (highly active antiretroviral therapy [HAART]). The method is based on the determination of adipocyte fatty acid binding protein A-FABP and, optionally, adiponectin in a sample of a HIV infected subject. If further information is wanted or needed, the method of the present invention may comprise the determination of C-reactive protein CRP.

**Description**

The present invention relates to a method of diagnosing metabolic alterations potentially leading to a cardiac complication or death in a human immunodeficiency virus (HIV) infected subject. Preferably, the HIV infected subject is under an antiretroviral therapy, in general an active antiviral therapy including administration of combinations of antiretroviral drugs for HIV therapy (highly active antiretroviral therapy [HAART]). The method is based on the determination of adipocyte fatty acid binding protein (A-FABP) and, optionally, adiponectin in a sample of a HIV infected subject. If further information is wanted or needed, the method of the present invention may comprise the determination of C-reactive protein CRP. The method of the present invention may even comprise the step of determining a cardiac troponin in the individual. Moreover, the present invention pertains to a method of predicting the risk of a HIV infected subject to suffer from a cardiac complication or death, based on the determination of the above-referenced markers/peptides. Moreover, the present invention relates to a method of monitoring the treatment of a HIV infected subject, and/or recommending or deciding on the adaptation of the treatment of a HIV infected subject. Further, devices and kits are encompassed for carrying out said methods, as well as the uses of the methods and devices and of binding ligands, in particular antibodies, in the methods of the present invention.

It is known to the person skilled in the art that metabolic syndrome (which often precedes diabetes type 2) and diabetes type 2 are in general associated with an increased risk of suffering from a cardiac complication. One component of the metabolic syndrome is obesity characterised by an increased body mass index or waist to hip ratio. Metabolic syndrome and type 2 diabetes are associated with increased risk of cardiovascular complications such as myocardial infarction or stroke.

Several hormones are involved in the development of the metabolic syndrome. Up to date, however, it is not clear which causal relationship exists between the various hormones or peptides - which may be upregulated or downregulated - and the occurrence of cardiac complications. A major hormone is adiponectin. Adiponectin is the most abundant adipokine secreted by adipocytes. Adipocytes are endocrine secretory cells which release free fatty acids and produce, in addition to adiponectin, several cytokines such as tumor necrosis factor (TNF) alpha, leptin, and interleukins.

A-FABP is one of the most abundant proteins in mature adipocytes. It is believed that in addition to its role in lipid metabolism and insulin sensitivity, A-FABP is a central player in mediating obesity-related vascular disease, primarily by inducing insulin resistance and potentiating lipids-induced inflammation. Circulating levels of A-FABP are closely correlated with several key features of the metabolic syndrome, including adverse lipid profiles, hyperglycemia, and hypertension. Second, the circulating level of A-FABP is a predictor of diabetes. See Xu et al, Advances in Pharmacology, Volume 60, 2010, pp 229-255 and literature cited therein.

The data in the scientific literature regarding the relationship between the A-FABP and the adiponectin level and the outcome are, however, very inconsistent. While some groups report that subjects with low adiponectin levels are at reduced risk of suffering from cardiac complications, other groups report that subjects with increased amounts of adiponectin are at increased risk of cardiac complications. Similar considerations apply for A-FABP.

It can, however, be said that the association of metabolic syndrome and of type 2 diabetes to cardiac complications is well established.

In HIV infected individuals, fat redistribution processes, e.g. obesity, are also a frequent finding in particular if the individuals are on antiretroviral therapy. Both mortality and morbidity for infections associated with HIV infection have been dramatically reduced by antiretroviral therapy. Fat redistribution processes in HIV are frequently associated with the use of antiretroviral drugs, in particular when combinations of two or more antiretroviral drugs are used for HIV therapy (highly active antiretroviral therapy [HAART]). The syndrome which is called HIV lipodystrophy or HIV lipodystrophic syndrome. HIVLD is associated with central fat accumulation but in contrast to the metabolic syndrome also associated with peripheral wasting such as decreased subcutaneous fat of the limbs together with prominent veins, loss of buttock fat and facial atrophy. There is also fat accumulation in the dorso cervical region which is associated with the term buffalo neck (Sweeney L.L. et al AIDS 2007: 21 895 - 904). While full blown HIVLD is clearly visible, this may not be the case in early HIVLD. Moreover metabolic syndrome and HIVLD - which are different pathological states which may not be mixed up with each other may occur in the same person. Disturbances of fat redistribution are generally associated with glucose or lipid metabolic abnormalities. It was shown that the fat redistribution was consistently associated with certain dysmetabolic features, for example, insulin resistance, glucose intolerance, hypertriglyceridaemia and low high-density lipoprotein (HDL) cholesterol. Insulin resistance, reflected by fasting hyperinsulinemia and inappropriate insulin response to standard glucose challenge, is frequently observed in HIV-infected patients, although an overt diabetes is less frequent.

While the link between HIV infected subjects, in particular HIV infected subjects under antiretroviral therapy, and cardiac complications and death is not fully clear, it is however established that a risk for the occurrence of a cardiac complication exists at least in some of these subjects. It is not clear if the risk is associated to the type of antiretroviral therapy, or depends from other variables.

As for metabolic syndrome, several publications can be found in the literature on the levels of adiponectin and the link to cardiac complications in HIV infected subjects which may be on antiretroviral therapy or not and may have developed so-called HIV lipodystrophy HIVLD or not.

Specific antiretrovirals have been associated with risk of myocardial infarction, diabetes mellitus and development of metabolic and morphologial disturbances including dyslipidemia, insulin resistance and body-fat change. These changes differ between drug classes. See Randell et al, Antiviral Therapy 2010, 16, p 227 - 233 and literature cited therein.

Protease inhibitors are known to inhibit the intrinsic activity of glucose transporter 4 (GLUT 4), to alter degradation of sterol-regulatory-element-binding protein 1 (SREBP-1) and to alter function of low density lipoprotein-receptor-related protein (LRP) (Sweeney L.L. et al AIDS 2007: 895 - 904). Moreover nucleoside reverse transcriptase analoges inhibit DNA polymerase gamma an enzyme essential for mitochondrial DNA replication, this resulting in mitochondrial DNA depletion, adipocyte death and lipoatrophy (Sweeney et al, l.c.).

Metabolic alterations can be observed in patients receiving antiretroviral therapy in the absence of obesity, hypertension or hyperlipidemia, contrary to subjects suffering from the metabolic syndrome. See Haider et al, Clinical Pharmacology & Therapeutics, vol. 81, no 4, p. 580 - 585 and literature cited therein.

The metabolic effects of antiretroviral therapy can be expected to increase the risk of cardiac complications. The risk of myocardial infarction (MI) appears to increase with longer exposure to antiretroviral therapy (Friis-Moller et al. N Engl J Med 2007; 356:1723-1735), although traditional risk factors still remain the main determinants of cardiac risk in HIV infected adults (Sterne JA, et al. J Intern Med 2007; 261:255-267). There are also suggestions that HIV itself increases the risk for cardiac complications.

Individuals at increased risk of cardiac complications require early and more intense workup regarding so far undetected cardiovascular abnormalities and may require treatment although still being asymptomatic.

A further problem in the present context resides in the fact that metabolic alterations in HIV are hard to recognize and diagnose in the early state, and that they can often not be distinguished from metabolic syndrome, or only at a later stage of the disease. In subjects who suffered from metabolic syndrome prior to the initiation of antiretroviral therapy, the typical fat redistribution pattern attributed to metabolic syndrome is often no longer existant, as a consequence of the peripheral wasting observed in metabolic alterations in subjects under antiretroviral therapy.

As described above, cardiac complications often develop gradually and patients may not show symptoms for a long time. Such patients do not receive appropriate treatment early in the course of the disease where therapeutic interventions may stop or even reverse the development of said conditions. In this respect, it has to be taken into consideration that antiretroviral therapy does not eliminate the HIV viruses from the body of the subject and antiretroviral therapy is a long lasting therapy, in general lasting over the entire lifetime of the concerned subject. Thus, it is highly desirable to identify patients with an increased risk of said diseases as early as possible in order to take preventive measures. Even if such measures are not possible, a reliable and simple prediction of the risk of a patient of suffering cardiac allows for dose monitoring of high-risk patients so that these patients can receive appropriate treatment at the onset of the disease.

The conventional diagnostic techniques for diagnosing and predicting cardiac complications include electrocardiographic and echocardiographic measurements, analysis of symptoms and previous medical history of the subject, such as chest pain, and analysis of some clinical parameters. Recently, these conventional techniques have been further strengthened by the analysis of biomarkers.

The major goal of HAART is to reduce viral load to undetectable levels HIV-RNA (below 0,05 copies per microliter) which is associated with improvement of immune function as assessed by the number of CD 4 positive cells and lack of AIDS defining conditions. While this is the major goal of treatment, current recommendations for the treatment of HIV infection require to consider viral resistance to antiviral drugs, pill burden, dosing schedule, tolerability profile and comorbid conditions like cardiovascular or renal disease as well as with the hepatitis B and C viruses (Zolopa A.R. Antiviral Research 2010: 85: 241 - 244). To expand this goal also conditions that are at increased risk to develop such conditions require consideration, specifically as patients with HIV get older and reach an age at which age related conditions become more frequent, such a prominent condition is a cardiovascular conditions which is the leading cause of death worldwide.

Thus there is an urgent need to have methods to identify such risk factors early and specifically those that are related to HIV drug therapy. There is, furthermore, an urgent need to have methods at hand to distinguish between conditions and/or drugs, in particular antiretroviral drugs, which are connected with high risk factors and those with no or low risk factors.

This object is attained by the methods, uses and devices as specified herein below.

Accordingly, the present invention provides a method of diagnosing metabolic alterations potentially leading to a cardiac complication or death, in a HIV-infected subject, preferably a HIV-infected subject receiving an antiretroviral therapy, based on the comparison of the amount of A-FABP, preferably determined beforehand in a sample of the subject, to at least one reference amount.

The method comprises at least one of the following steps: a) determining the amount of A-FABP or a variant thereof in a sample of the subject; b) comparing the amount to reference amount; c) diagnosing metabolic alterations, based on the comparison carried out in step b).

Thus, in one aspect of the present invention, there is provided a method of diagnosing metabolic alterations in a HIV infected subject potentially leading to a cardiac complication or death, comprising the following steps:

a) determining the amount of A-FABP or a variant thereof in a sample of the subject
b) comparing the amount to reference amount
c) diagnosing the metabolic alterations, based on the comparison carried out in step b).

In a further aspect, the present invention provides a method of predicting the risk of a HIV-infected subject to suffer from a cardiac complication or death, based on the comparison of the amount A-FABP, preferably determined beforehand in a sample of the subject, to at least one reference amount.

The method comprises at least one of the following steps: a) determining the amount of A-FABP or a variant thereof in a sample of the subject; b) comparing the amount to reference amount; c) predicting the risk, based on the comparison carried out in step b).

Thus, in one aspect of the present invention, there is provided a method of predicting the risk of a HIV infected subject to suffer from a cardiac complication or death, comprising the following steps:

a) determining the amount of A-FABP or a variant thereof in a sample of the subject
b) comparing the amount to reference amount
c) predicting the risk, based on the comparison carried out in step b)..

The subject, preferably and in most cases, is a HIV-infected subject to whom an antiretroviral therapy is administered. The antiretroviral therapy is preferably a highly active antiretroviral therapy HAART.

In a preferred embodiment of the present invention, the amount of adiponectin or a variant thereof is determined in the sample, further to the amount of A-FABP or a variant thereof, and metabolic alterations are diagnosed and/or the risk is predicted based on the comparison of the marker amounts with reference amounts

In further preferred embodiment of the present invention, the amount of C-reactive protein CRP or a variant thereof is determined in the sample further to the amount of A-FABP or a variant thereof and, optionally, adiponectin or a variant thereof, and the metabolic alterations are diagnosed and/or the risk is predicted based on the comparison of the marker amounts with reference amounts.

The method of the present invention, preferably, is an in vitro method. Moreover, it may comprise steps in addition to those explicitly mentioned above. For example, further steps may relate to sample pre-treatments or evaluation of the results obtained by the method. The method of the present invention may be also used for monitoring, confirmation, and subclassification of a HIV infected subject, preferably a HIV infected subject receiving an antiretroviral therapy, more preferably receiving HAART, in respect to the said complications. The method may be carried out manually or assisted by automation. Preferably, step (a) and/or (b) and/or c) may in total or in part be assisted by automation, e.g., by a suitable robotic and sensory equipment for the determination in step (a) or a computer-implemented comparison in step (b) or a computer-implemented prediction in step c).

The terms "diagnosing" and "diagnosing metabolic alterations" as used herein refer to assessing and/or identifying if a HIV infected subject, preferably a HIV infected subject receiving an antiretroviral therapy, more preferably receiving HAART, suffers from an alteration or disturbance in his metabolism, and wherein the disturbance/alteration is associated with or leads to a cardiac complication or death; i.e. the disturbance/alteration is known to be often or even generally followed by a cardiac complication and/or death (i.e. mortality), or the disturbance/alteration is known to often or even generally leads to a cardiac complication and/or death, within a defined time frame (prediction frame) in the future. The cardiac complication may follow the metabolic alterations/disturbances, or the cardiac complication may coincide with the metabolic disturbance/alteration, or the cardiac complication may be caused by the metabolic disturbance/alteration.

The term "leading to" as used herein encompasses a temporal relationship, an association (coincidence), or a causal relationship between two events. Here, i.e. in the case of a metabolic alteration in the subject which potentially results in a cardiac complication and/or death, the term is meant to convey that (i) the metabolic alteration is followed by a cardiac complication and/or death, (temporal relationship), or (ii) the metabolic alteration is associated in the sense of coinciding with a cardiac complication/death, or (iii) the metabolic alteration causes the cardiac complication/death.

The term "potentially" means that a certain probability or risk exists according to which an individual suffering from the said metabolic alteration/disturbance will, later on, suffer from a cardiac complication and/or death. Accordingly, the term "potentially" expresses the risk or probability according to which at least one of the above-cited outcomes will occur within a certain time frame, as defined in the following paragraphs. The term "metabolic alteration" or "metabolic disturbance" as used in the context of the present invention relates to changes in the lipid and/or glucose metabolism of the respective individual who has a HIV infection and who is preferably treated by an antiretroviral therapy, in particular HAART, vis-à-vis the same individual who is healthy.

The term "predicting" and "predicting the risk" as used herein refers to assessing the probability according to which a HIV infected subject, preferably a HIV infected subject receiving an antiretroviral therapy, more preferably receiving HAART, will suffer from a cardiac complication or death (i.e. mortality) within a defined time frame (prediction frame) in the future. The mortality may be caused by the cardiac complication, or death may be the consequence of the cardiac complication. The prediction frame is an interval in which the subject will develop one or more of the said complications according to the predicted probability. The prediction frame may be the entire remaining lifespan of the subject upon analysis by the method of the present invention. Preferably, however, the prediction frame is an interval of one month, six months or one, two, three, four, five or ten years after appearance of the HIV infection and/or the beginning of the antiretroviral therapy (more preferably and precisely, after the sample to be analyzed by the method of the present invention has been obtained).

As will be understood by those skilled in the art, such an assessment is usually not intended to be correct for 100% of the subjects to be analyzed. The term, however, requires that the assessment will be valid for a statistically significant portion of the subjects to be analyzed. Whether a portion is statistically significant can be determined without further ado by the Person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Preferably, the probability envisaged by the present invention allows that the prediction will be correct for at least 60%, at least 70%, at least 80%, or at least 90% of the subjects of a given cohort.

The terms "subject", "patient" or "individual" may be used interchangeably in the context of the present invention and refer to the same. The term "subject" is preferred. The term "subject" as used herein relates to animals, preferably mammals, and, more preferably, humans. However, it is envisaged in accordance with the aforementioned method of the present invention that the subject shall suffer from a HIV infection. The subject thus exhibits the signs of clinical disorders according to category B or C which are known to the person skilled in the art and which are laid out hereinafter. More details can be found e.g. under www.merck.com/mmpe/index.html. under "Human Immunodeficiency Virus (HIV)".

Human immunodeficiency virus (HIV) infection results from 1 of 2 similar retroviruses (HIV-1 and HIV-2) that destroy CD4$^+$ lymphocytes and impair cell-mediated immunity, which increases the risk of certain infections and cancers. An initial infection may cause nonspecific febrile illness. The risk of subsequent manifestations which are related to immunodeficiencies are proportional to the level of CD4 lymphocytes. Manifestations can range from asymptomatic carriage to AIDS, which is defined by serious opportunistic infections or cancers or a CD4 count of < 200/$\mu$L. HIV infection can be diagnosed by antibody or antigen testing.

AIDS is defined as HIV infection that leads to any of various disorders in clinical category B or C of HIV infection. The disorders in categories B and C are serious opportunistic infections, certain cancers, such as Kaposi's sarcoma and non-Hodgkin lymphoma, to which defective cell-mediated immunity predisposes, and neurologic dysfunction. For example, category B includes cervical dysplasia (moderate or severe) or cervical carcinoma in situ, constitutional symptoms, such as fever or diarrhea lasting for more than 1 month, oral hairy leukoplakia, herpes zoster (shingles), listeriosis and pelvic inflammatory disease. Type C, for example, includes candidiasis of bronchi, trachea, lungs, or esophagus, cervical cancer, HIV-related encephalopathy, Kaposi's sarcoma and Burkitt's lymphoma. It is to be understood, however, that the subjects according to the present invention do not have entered into category B or C, i.e. the subjects are asymptomatic in respect to HIV infection.

The main consequence of HIV infection is damage to the immune system, specifically loss of CD4$^+$ lymphocytes, which are involved in cell-mediated and, to a lesser extent, humoral immunity.

Antibodies to HIV are measurable usually within a few weeks after primary infection; however, antibodies cannot fully control HIV infection.

Risk of AIDS, death, or both is predicted by the CD4 count in the short term and by plasma HIV RNA level in the longer term. For every 3-fold increase in viral load, mortality over the next 2 to 3 years increases about 50%. HIV-associated morbidity and mortality vary by the CD count, with the most deaths from HIV-related causes occurring at counts of < 50/$\mu$L. However, with effective treatment, the HIV RNA level decreases to undetectable levels, CD4 counts often increase dramatically, and risk of illness and death falls.

Treatment of HIV infections is carried out by administration of antiretroviral drugs, preferably combinations of antiretroviral drugs. Because adequate antiretroviral therapy can cause significant long-term morbidity, it is not recommended for everyone. Current indications include a CD4 count of < 350/$\mu$L and HIV RNA level of > 55,000 copies/mL. Use of antiviral drugs aims to reduce the plasma HIV RNA level and restore the CD4 count. Often, potent combinations of antiretroviral drugs (usually 3 or four) are used for HIV therapy (highly active antiretroviral therapy HAART) The lower the pretreatment CD4 count and the higher the HIV RNA level, the less likely treatment is to succeed, but marked improvement is observed even in patients with advanced immunosuppression.

HAART aims to suppress viral replication to undetectable levels. It is, however, known that patients beginning HAART sometimes deteriorate clinically, despite increasing CD4 counts, because of an immune reaction to subclinical opportunistic infections or to residual microbial antigens after successful treatment of opportunistic infections.

It is also known that even adequate antiretroviral therapy can cause cardiac complications or death and can cause significant long-term morbidity. HAART aims to reduce the plasma HIV RNA level and restore the CD4 count (immune restoration or reconstitution). The lower the pretreatment CD4 count and the higher the HIV RNA level, the less likely treatment is to succeed, but marked improvement is observed even in patients with advanced immunosuppression.

Several classes of antiretroviral drugs are used in antiretroviral therapy and HAART. There are 5 classes of antiretrovirals which are specified hereinafter.

Nucleotide reverse transcriptase inhibitors (nRTIs) competitively inhibit the HIV reverse transcriptase enzyme. Contray to NRTIs (see below), they do not require initial phosphorylation. Non-limiting examples include Truvada® (Tenofovir and Emtricabine) and Viread® (Tenofovir)

Nucleoside reverse transcriptase inhibitors (NRTIs) are phosphorylated to active metabolites that compete for incorporation into viral DNA. They inhibit the HIV reverse transcriptase enzyme competitively and terminate synthesis of DNA chains. Non-limiting examples include Viramune® (Neviparin), Kivexa® (Abacavir and Lamivudine), Combivir® (Idovudine and Lamivudine), Retrovir® (Zidovudine), Videx® (DDI), Epivir® (Lamivudine), Ziagenavir® (Abacavir).

Non-nucleoside reverse transcriptase inhibitors (NNRTIs) bind directly to the reverse transcriptase enzyme. Non-limiting examples include Sustiva® (Efavirenz), Emtriva® (Emtricabine).

Protease inhibitors (PIs) inhibit the viral protease enzyme that is crucial to maturation of immature HIV virions after they bud from host cells. Non-limiting examples include Crixivan® (Indinavir), Kaletra® (Lopinavir and Ritonavir), Reyataz® (Atzanavir), Telzir® (Fosanprenavir), Invirase® (Saquinavir), Prezistza® (Duranavir).

Further known are entry inhibitors (EIs), sometimes called fusion inhibitors, they interfere with the binding. Non-limiting examples include Fuceon® (Enfuvirtid).

Integrase inhibitors prevent HIV DNA from being integrated into human DNA. Non-limiting examples include Insentress® (Raltegravir).

Lipodystrophy or lipodystrophy syndrome has been commonly recognized in patients receiving HAART. In the context of the present invention, lipodystrophy will be referred to as HIV related lipodystrophy HIVLD. This syndrome associates at various levels disturbances in fat redistribution, dyslipidemia, and insulin resistance. The main clinical features of fat redistribution are the loss of subcutaneous fat in the face, limbs, and buttocks, accompanied by a central fat accumulation in the abdomen, breast, and over the dorsocervical spine ("buffalo hump"), and sometimes lipomas. Only a few patients have a pure fat atrophy; a second group displays visceral adiposity alone, whereas most patients combine both peripheral lipoatrophy and visceral adiposity. The changes in body composition may contribute to noncompliance with or discontinuation of HAART.

Disturbances of fat redistribution are associated with glucose or lipid metabolic abnormalities. Insulin resistance and inappropriate insulin response to standard glucose challenge is frequently observed in HIV infected patients. An overt diabetes is less frequent. Dyslipidemia includes elevated serum triglycerides, LDL- and VLDL-cholesterol, and decreased HDL-cholesterol. The prevalence HIVLD among HIV infected individuals varies widely, with estimations ranging from 20% to 84%. The mechanisms responsible for the development of the HIVLD remain largely unknown, but the etiology is likely multifactorial. It is thought that various components contribute, including HAART, genetic factors, and the HIV virus itself, leading to fat redistribution, dyslipidemia, and insulin resistance. The occurrence of HIVLD temporally after the use of HAART has raised the possibility that the LDS could be related to drug toxicity.

Protease inhibitor (PI) use appears to be associated strongly with visceral adiposity and metabolic side effects. Nucleoside reverse transcriptase inhibitor (NRTI) therapy is also believed to be involved in the development of peripheral fat loss, although it appears to have less effect on lipid and glucose metabolism. Administration of a combined treatment by PI and NRTI more frequently induce changes in body composition than the use of PI or NRTI alone.

The subject-matter of the present invention, however, is not intended for the diagnosis or risk prediction of subjects already suffering from HIVLD or clearly recognizable HIVLD, but is drawn to the diagnosis of metabolic alterations potentially leading to a cardiac complication or death or, respectively, the prediction of the risk of a cardiac complication in a subject who does not suffer from HIVLD. It is to be understood that the individual may not suffer from HIVLD at all. It is also possible, in the context of the present invention, that the subject may suffer from forms of HIVLD which are not clearly to be diagnosed by standard methods (preferably visible inspection). This includes early forms of HIVLD.

Preferably, the subject according to the present invention does not suffer from metabolic syndrome/and or diabetes type 2. It is known to the person skilled in the art that the amounts of A-FABP and/or adiponectin are often elevated (A-FABP) and, respectively, diminished (adiponectin) as a consequence of metabolic syndrome/type 2 diabetes, when compared to healthy individuals, and to a higher extent than in HIV infected individuals under antiretroviral/HAART therapy. Accordingly, although it may be possible to diagnose metabolic alterations after antiretroviral therapy/HAART by the amount of A-FABP/HAART, this may in some cases or often not be possible due the higher relative increase of A-FABP/higher relative diminuation of adiponectin in metabolic syndrome/type 2 diabetes.

In the context of the present invention, the inventors could find that on the average, A-FABP levels in populations of HIV infected subjects, under antiretroviral therapy, are not elevated in respect to normal, healthy subjects. It was even found that on the average in the above population of HIV infected subjects, A-FABP levels were even slightly lower than in normal, healthy subjects. Furthermore cardiac troponin levels indicated that on the average, A-FABP levels in HIV infected subjects, whether or not on antiretroviral therapy, are not related to an increased risk of suffering from a cardiac complication or death in respect to normal, healthy subjects. Normal, healthy subjects did not display lower troponin levels than HIV infected subjects (see examples).

A detailed analysis of the data which were collected in the framework of the clinical studies underlying the present invention and carried out by the inventors, however, clearly showed a correlation between elevated A-FABP levels and an increased risk of suffering from a cardiac complication. HIV infected subjects having elevated A-FABP levels carried a higher risk of suffering from a cardiac complication or death than those HIV infected subjects not having elevated A-FABP levels. The high risk of suffering from the above-cited events was shown by significantly higher amounts of cardiac troponins, reaching those levels which are known to the person skilled in the art as being indicative for a clear risk of suffering from a cardiac adverse event and/or death.

This is a surprising finding in particular in consideration of the fact that in non HIV infected subjects suffering from metabolic syndrome (which are known to be risk-bearing in respect to adverse cardiac complications), A-FABP levels are highly elevated relative to normal, healthy subjects. The same holds true for diabetes type 2 patients where, on the average, A-FABP levels amount to about the same levels as in metabolic syndrome subjects. Troponin values are, however, clearly more elevated in type 2 diabetes subjects, compared to metabolic syndrome subjects, showing a clearly higher risk for diabetes subjects to suffer from a cardiac complication.

Troponin levels in HIV infected subjects, on the average, amount to those found in metabolic syndrome patients (which here are related to an increased cardiovascular risk). However, average A-FABP amounts in HIV infected subjects, in particular those receiving antiretroviral therapy, are clearly lower than in subjects suffering from metabolic syndrome (and, of course, also lower than in diabetes type 2 subjects

In a further embodiment of the present invention, the amount of a cardiac troponin, preferably troponin T or I, is additionally determined in the subject in whom A-FABP and, as the case may be, adiponectin and/or CRP have been determined. On the one hand, this permits a confirmation of the diagnosis/prediction according to the method of the present invention, and shows that antiretroviral treatment has already affected the cardiac system of the subject. On the other hand, if A-FABP is elevated (and adiponectin is decreased) and the cardiac troponin is not elevated, this means that the metabolic alteration connected to the rise in A-FABP has not yet affected the cardiac system.

It is known that a Troponin T test with a sensitivity of 3 pg/ml detected Troponin T concentration not only in patients with acute coronary syndrome and myocardial infarction but also in patients with clinically stable chronic artery disease. (EP 07114174.1 / 1 890 154). Using Troponin T quartiles it could be demonstrated that Troponin T concentrations correlated with NT-proBNP levels and, thus, with the extent of heart failure. In addition the level of sensitive Troponin T has been shown to be linked to future heart failure and its frequency (Omland, Journal of Internal Medicine 268 (2010), pages 207-217).

In the context of the present invention, it was found that some of the above-specified antiretroviral drugs and/or antiretroviral drug classes trigger metabolic alterations in a higher extent than other antiretroviral drugs and/or antiretroviral drug classes, as determined by the amounts of the markers used in the present context (see also Example 2).

The following antiretroviral drug classes and drug class combinations are the most powerful triggers of metabolic alterations potentially leading to a cardiac complication: nRTIs; PIs + NRTIs; NNRTIs + NRTIs; PIs + NRTIs + nRTIs; (IV; I + III; II + III; I + III + IV)

The following antiretroviral drug classes and drug class combinations are the least powerful triggers of metabolic alterations potentially leading to a cardiac complication:

PIs, NNRTIs + nRTIs ; NNRTIs + NRTIs + nRTIs; NRTIs; NRTIs + nRTIs (I; II + III + IV ; III ; III + IV)

The roman numbers have the following meaning: Nucleotide reverse transcriptase inhibitors (nRTIs) IV; Nucleoside reverse transcriptase inhibitors (NRTIs) III; Non-nucleoside reverse transcriptase inhibitors (NNRTIs) II; and Protease inhibitors (PIs) I.

Accordingly, in a preferred embodiment, the HIV infected subject is preferably a subject who is treated with an antiretroviral drug class or a drug class combinations comprising or consisting of nRTIs, PIs and NRTIs, NNRTIs and NRTIs, and PIs and NRTIs and nRTIs; (i.e. (IV, I and III, II and III, I and III and IV). These individuals encounter the highest risk for suffering from metabolic alterations leading to a cardiac complication and, accordingly, a change of the treatment/drug combination should be considered, towards drug/drug combinations not triggering metabolic alterations or triggering them to a lesser extent.

In a further preferred embodiment, the HIV infected subject is preferably a subject who is treated with an antiretroviral drug class or a drug class combinations comprising or consisting of PIs, NNRTIs and nRTIs ; NNRTIs and NRTIs and nRTIs; NRTIs ; NRTIs and nRTIs (I; II and III and IV ; III ; III and IV). These individuals encounter a lower risk for suffering from metabolic alterations and, accordingly, a change of the treatment/drug combination needs not to be considered.

The term "cardiac complication" or "adverse cardiac complication" as used herein refers to any disorder of the cardiac system. These are known to the person skilled in the art.

The term includes, on the one hand, cardiovascular complications. Cardiovascular complications can be separated into chronic cardiovascular complications and acute cardiovascular complications. In cardiovascular complications, heart tissue (preferably pericardium, myocardium, or cardiac valves) and heart vessels (coronary vessels) are involved in the disease. Chronic cardiovascular complications or diseases are e.g. cardiac ischemia and coronary artery diseases CAD. These chronic complications /diseases can occur in stable patients, i.e. those not suffering from an acute deterioration of their physiological status. In the present invention, the term "cardiovascular complications" is preferably drawn to acute cardiovascular events (i.e. events connected to a sudden deterioration of the subject's physiological status). Acute cardiovascular events are, preferably, stable angina pectoris (SAP) or acute coronary syndrome (ACS). ACS patients can show unstable angina pectoris (UAP) or myocardial infarction (MI). MI can be an ST-elevation MI (STEMI) or a non-ST-elevation MI (NSTEMI). NSTE-ACS as used herein encompasses UAP and NSTEMI. The occurring of an MI can be followed by a left ventricular dysfunction (LVD), development of heart failure or even mortality. Further preferred cardiovascular events encompass cardiac brady- or tachyarrhythmias including sudden cardiac death and stroke (cerebrovascular events or accidents).

The term "cardiac complication" also refers to heart failure HF, in the context of the present invention. Heart failure is a complex clinical syndrome that can result from any structural or functional cardiac disorder that impairs the ability of the ventricle to fill with or eject blood and to ensure the body's metabolic needs for supply with blood/oxygen. In such cases, the body tries to compensate lack of supply by structural changes of the myocardium (e.g. fibrosis, apoptosis, necrosis) aiming at maintaining the required supply. First structural changes to the myocardium are, in general, reversible changes but which, when untreated, turn to non reversible permanent changes which finally lead to chronic HF with the final stage of terminal HF. HF is classified into various degrees of severity.

One classification is the so-called NYHA (New York Heart Association) classification. Heart failure patients are classified into NYHA classes I, II, III and IV. A patient having heart failure has already experienced structural and functional changes to his pericardium, myocardium, coronary circulation or cardiac valves. He will not be able to fully restore his health, and is in need of a therapeutical treatment. Patients of NYHA Class I have no obvious symptoms of cardiovascular disease but already have objective evidence of functional impairment. Patients of NYHA class II have slight limitation of physical activity. Patients of NYHA class III show a marked limitation of physical activity. Patients of NYHA class IV are unable to carry out any physical activity without discomfort. They show symptoms of cardiac insufficiency at rest.

This functional classification is supplemented by the more recent classification by the American College of Cardiology and the American Heart Association (see J. Am. Coll. Cardiol. 2001;38;2101-2113, updated in 2005, see J. Am. Coll. Cardiol. 2005;46;e1-e82). 4 stages A, B, C and D are defined. Stages A and B are not HF but are considered to help identify patients early before developing "truly" HF. Stages A and B patients are best defined as those with risk factors for the development of HF. For example, patients with coronary artery disease, hypertension, or diabetes mellitus who do not yet demonstrate impaired left ventricular (LV) function, hypertrophy, or geometric chamber distortion would be considered stage A, whereas patients who are asymptomatic but demonstrate LV hypertrophy (LVH, a phenomenon in which the walls of the ventricles thicken) and/or impaired LV function would be designated as stage B. Stage C then denotes patients with current or past symptoms of HF associated with underlying structural heart disease (the bulk of patients with HF), and stage D designates patients with truly refractory HF.

The term "heart failure" as used herein relates to an impaired systolic and/or diastolic function of the heart being accompanied by overt signs of heart failure as known to the person skilled in the art. Preferably, heart failure referred to herein is also chronic heart failure. Heart failure according to the present invention includes overt and/or advanced heart failure. In overt heart failure, the subject shows symptoms of heart failure as known to the person skilled in the art.

The term "heart failure" as used herein refers to stages C and D of the ACC/AHA classification; in these stages, the subject shows typical symptoms of heart failure. i.e. the subject is not apparently healthy. The subject having heart failure and being classified into stage C or D has undergone permanent, non reversible structural and/or functional changes to his myocardium, and as a consequence of these changes, full health restoration is not possible. A subject having attained stage C or even D of the ACC/AHA classification cannot go back to stage B or even A. "Heart failure" as used herein also refers to stages I, II, III and IV, preferably II, III and IV of the NYHA classification.

Manifestations (symptoms) of HF are dyspnea and fatigue and fluid retention, which may lead to pulmonary congestion and peripheral edema, typical signs on the physical examination are edema and rales. There is no single diagnostic test for HF because it is largely a clinical diagnosis that is based on a careful history and physical examination.

The clinical syndrome of HF may result from disorders of the pericardium, myocardium, endocardium, or great vessels

Heart failure can occur after or be preceded by an acute cardiovascular event, frequently MI, see beforehand. However, heart failure can also occur after or be caused by an infection, administration of cardiotoxic drugs, hormones like erythropoietin, or, in many cases, by arterial hypertension. Heart failure can also occur after or be caused by a chronic cardiovascular disease, an ischemic cardiovascular disease and/or by coronary artery disease CAD, e.g. LVD or diastolic dysfunction. In sum, the term heart failure as used herein refers to any kind of heart failure, irrespective of the pathological states preceding it or by which it is caused.

Preferred cardiac complications in the sense of the present invention are chronic cardiovascular diseases, ischemic cardiovascular diseases, coronary artery disease CAD and heart failure, in particular heart failure.

The terms "death" and "mortality" as used herein relate to mortality from any cause, preferably, from a cardiac complication or cardiovascular complication or from heart failure. Also, mortality can also refer to the death rate or the ratio of number of deaths to a given population of subjects.

The expression "predicting the risk of a cardiac complication and/or death/mortality" as used herein means that if the subject to be examined by the methods of the present invention is allocated either into the group of subjects of a population having a normal, i.e. average, risk for developing a cardiac complication or mortality, or into a group of subjects having a elevated risk, or into a group having a reduced risk. An elevated risk as referred to in accordance with the present invention, preferably, means that the risk of developing a cardiac complication or the risk of mortality within a predetermined prediction frame is elevated significantly (i.e. increased significantly) for a subject with respect to the average risk for a cardiac complication or cardiac mortality in a population of subjects (with the metabolic syndrome). A reduced risk as referred to in accordance with the present invention, preferably, means that the risk of developing a cardiac complication or the risk of mortality within a predetermined prediction frame is reduced significantly for a subject with respect to the average risk for a cardiac complication or cardiac mortality in a population of HIV infected subjects. Particularly, a significant increase or reduction of a risk is an increase or reduction or a risk of a size which is considered to be significant for prognosis, particularly said increase or reduction is considered statistically significant. The terms "significant" and "statistically significant" are known by the person skilled in the art. Thus, whether an increase or reduction of a risk is significant or statistically significant can be determined without further ado by the person skilled in the art using various well known statistic evaluation tools.

**A-FABP**

Adipose tissue is a critical regulator of vascular function, which until recently has been virtually ignored. Almost all blood vessels are surrounded by perivascular adipose tissue, which is actively involved in the maintenance of vascular homestasis by producing "vasocrine" signals such as adipocines. Adiponectin and adipocyte fatty acid binding protein (A-FABP), both of which are major adipocines predominantly produced in adipose tissues, have recently been shown to be pivotal modulators of vascular function.

The term "adipocyte fatty acid binding protein" (A-FABP) relates to a polypeptide being an adipocyte type fatty acid binding protein and to a variant thereof. For a sequence of human A-FABP, see e.g. Baxa et al.; Biochemistry. 1989 Oct 31;28(22):8683-90, or Protein: P15090

A-FABP, also termed aP2 and FABP4, is one of the most abundant proteins in mature adipocytes (Makowski & Hotamisligil, The Journal of Nutrition 2004: 134: 2464S - 2468S). Levels of A FABP are closely related to key features of the metabolic syndrome including increased serum triglycerides and LDL cholecterol, hyperglycemia and hypertension (Hsu X. et al,J al Circulation Journal 2010: 327 - 331). Baseline levels of A-FABP were also found to predict the development of the metabolic syndrome during a 5 year follow up period (Xu A. et al Circulation 2007: 115: 1537 - 1543). A-FABP is also a strong predictor for future type 2 diabetes mellitus independently of the traditional risk factors (TSO a A et al Diabetes Care 2007: 30: 2557 - 2672). In addition, serum levels of A-FABP augment as the numbers of stenotic vessels increase from normal to three vessel disease, suggesting an etiological role of A-FABP in coronary artery disease (Rhee RE. et al Europ J Endocrinology 2009: 160: : 165 - 172). The underlying mechanism is the detrimental role of A-FABP in vascular dysfunction and atherosclerosis, mainly by acting as a lipid sensor to transmit toxic lipids-induced vascular inflammation through induction of endplasmatic stress ( Xu A. et al, Advances in Pharmacology 2010: 60: 229 - 255).

A number of FABP inhibitors have been identified, including carbazole- and indole-based inhibitors, benzylamino-6-(trifluoromethyl) pyrimidin-4 (1H)inhibitors, and a biphenyl azole inhibitor (also known as BMS309403, developed by Bristol-Myers Squibb (Furuhashi M et al, Nature Reviews 2008: 7: 489 - 503).

The term "A-FABP" encompasses also variants of A-FABP, preferably, of human A-FABP. Such variants have at least the same essential biological and immunological properties as A-FABP, i.e. they bind free fatty acids and/or cholesterol and/or retinoids, and/or are involved in intracellular lipid transport, see Hotamisligil et al, Science 22 November 1996: Vol. 274 no. 5291 pp. 1377-1379. In particular, they share the same essential biological and immunological properties if they are detectable by the same specific assays referred to in this specification, e.g., by ELISA Assays using polyclonal or monoclonal antibodies specifically recognizing the A-FABP. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the human A-FABP, preferably over the entire length of human A-FABP. How to determine the degree of identity is specified elsewhere herein. Variants may be allelic variants or any other species specific homologs, paralogs, or orthologs. Moreover, the variants referred to herein include fragments of L-FABP or the aforementioned types of variants as long as these fragments have the essential immunological and biological properties as referred to above. Such fragments may be, e.g., degradation products of the A-FABP. Further included are variants which differ due to posttranslational modifications such as phosphorylation or myristylation. The term "A-FABP or a variant thereof", preferably, does not include heart FABP, brain FABP and liver FABP.

**Adiponectin**

Adiponectin is a polypeptide (one of several known adipocytokines) secreted by the adipocyte. In the art, adiponectin is frequently also referred to as Acrp30 and apM1. Adiponectin has recently been shown to have various activities such as anti-inflammatory, antiatherogenic, preventive for metabolic syndrome, and insulin sensitizing activities. Adiponectin is encoded by a single gene, and has 244 amino acids, its molecular weight is approximately 30 kilodaltons. The mature human adiponectin protein encompasses amino acids 19 to 244 of full-length adiponectin. A globular domain is thought to encompass amino acids 107 - 244 of full-length adiponectin. The sequence of the adiponectin polypeptide is well known in the art, and, e.g., disclosed in WO/2008/084003.

Adiponectin is the most abundant adipokine secreted by adipocytes. Adipocytes are endocrine secretory cells which release free fatty acids and produce, in addition to adiponectin, several cytokines such as tumour necrosis factor (TNF) alpha, leptin, and interleukins.

It is generally assumed that adiponectin sensitizes the body to insulin. Decreased adiponectin blood levels are observed in patients with diabetes and metabolic syndrome and are thought to play a key role in insulin resistance (see e.g. Han et al. Journal of the American College of Cardiology, Vol. 49(5)531-8).

Adiponectin associates itself into larger structures. Three adiponectin polypeptides bind together and form a homotrimer. These trimers bind together to form hexamers or dodecamers. Adiponectin is known to exist in a wide range of multimer complexes in plasma and combines via its collagen domain to create 3 major oligomeric forms: a low-molecular weight (LMW) trimer, a middle-molecular weight (MMW) hexamer, and high-molecular weight (HMW) 12- to 18-mer adiponectin (Kadowaki et al. (2006) Adiponectin and adiponectin receptors in insulin resistance, diabetes, and the metabolic syndrome. J Clin Invest. 116(7): 1784-1792; Rexford S. Ahima, Obesity 2006;14:242S-249S). Adiponectin has been reported to have several physiological actions, such as protective activities against atherosclerosis, improvement of insulin sensitivity, and prevention of hepatic fibrosis. Adiponectin as used herein, preferably, relates to total adiponectin, which encompasses low molecular weight adiponectin, mid molecular weight adiponectin and high molecular weight adiponectin. The terms high molecular weight adiponectin, low and mid molecular weight adiponectin and total adiponectin are understood by the skilled person. Preferably, said adiponectin is human adiponectin. Methods for the determination of adiponectin are, e.g., disclosed in US 2007/0042424 A1 as well as in WO/2008/084003. The amount of adiponectin is determined in a urine sample.

The adiponectin referred to in accordance with the present invention further encompasses allelic and other variants of said specific sequence for human adiponectin discussed above. Specifically, envisaged are variant polypeptides which are on the amino acid level preferably, at least 50%, 60%, 70%, 80%, 85%, 90%, 92%, 95%, 97%, 98%, or 99% identical, to human adiponectin. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad. Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human adiponectin as long as the said polypeptides have adiponectin properties, in particular insulin sensitizing properties.

Preferably, the adiponectin variants have adiponectin properties, preferably, including its ability to reduce adipose tissue which can be tested in known animal models.

Whether a variant of adiponectin is capable of reducing adipose tissue can be, e.g., tested as follows: The tested variant can be administered to adipocytes, to tissue comprising adipocytes or to an non-human animal. Preferably, a variant of adiponectin is capable of reducing adipose tissue, if the amount of fat comprised by said adipocytes, said tissue or said non-human animal is decreased as compared with control adipocytes, a control tissue or not-human animal which have not been contacted with said variant. Preferably, the non-human animal will be sacrificed after the determining whether said variant reduces adipose tissues (It is, thus, to be understood that such a determination is not deemed to be a method of treatment of the human or animal body).

**CRP**

CRP, herein also referred to as C-reactive protein, is an acute phase protein that was discovered more than 75 years ago to be a blood protein that binds to the C-polysaccharide of pneumococci. CRP is known as a reactive inflammatory marker and is produced by a distal organ (i.e. the liver) in response or reaction to chemokines or interleukins originating from the primary lesion site. CRP has five single subunits, which are non covalently linked and assembled as a cyclic pentamer with a molecular weight of approximately 110-140 kDa. CRP binds to phosphocholine expressed on the surface of dead or dying cells and activates the complement system via the C1Q complex.

The term "CRP" preferably also relates to variants of CRP. Preferably, CRP as used herein relates to human CRP. The sequence of human CRP is well known and disclosed, preferably, in Woo 1985, J. Biol. Chem. 260 (24), 13384-13388. The level of CRP is usually low in normal individuals but can rise 100- to 200-fold or higher due to inflammation, infection or injury (Ych 2004, Circulation 109:11-11-11-14). Preferably, the amount of CRP in a sample of a subject is determined by using CRP assays with a high sensitivity. The CRP determined by such assays is frequently also referred to as high sensitivity CRP (hsCRP). HsCRP assays are, e.g., used to predict the risk of heart disease. Suitable hsCRP assays are known in the art. A particularly preferred hsCRP assay in the context of the present invention is the Roche/Hitachi CRP (Latex) HS test with a detection limit of 0.1 mg/l.

Moreover, the CRP as referred to in accordance with the present invention further variants of said specific sequence for discussed above. It is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific CRP polypeptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific CRP polypeptides or the aforementioned types of variants as long as these fragments have the essential immunological and biological properties as referred to above. Such fragments may be, e.g., degradation products or splice variants of the CRP polypeptides. Further included are variants which differ due to posttranslational modifications such as phosphorylation or myristylation. The preferred biological/immunological function of CRP is to bind to phosphocholine.

**Cardiac troponins**

The term "cardiac Troponin" refers to all Troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac Troponin refers to Troponin T and/or Troponin I, and, most preferably, to Troponin T. It is to be understood that isoforms of Troponins may be determined in the method of the present invention together, i.e. simultaneously or sequentially, or individually, i.e. without determining the other isoform at all. Amino acid sequences for human Troponin T and human Troponin I are disclosed in Anderson, loc cit and Ferrieres 1998, Clinical Chemistry, 44: 487-493.

The term "cardiac Troponin" encompasses also variants of the aforementioned specific Troponins, i.e., preferably, of Troponin I, and more preferably, of Troponin T. Such variants have at least the same essential biological and immunological properties as the specific cardiac Troponins. In particular, they share the same essential biological and immunological properties if they are detectable by the same specific assays referred to in this specification, e.g., by ELISA Assays using polyclonal or monoclonal antibodies specifically recognizing the said cardiac Troponins. Moreover, it is to be understood that a variant as referred to in accordance with the present invention shall have an amino acid sequence which differs due to at least one amino acid substitution, deletion and/or addition wherein the amino acid sequence of the variant is still, preferably, at least about 50%, at least about 60%, at least about 70%, at least about 80%, at least about 85%, at least about 90%, at least about92%, at least about95%, at least about 97%, at least about 98%, or at least about 99% identical with the amino sequence of the specific Troponin. Variants may be allelic variants or any other species specific homologs, paralogs, or orthologs. Moreover, the variants referred to herein include fragments of the specific cardiac Troponins or the aforementioned types of variants as long as these fragments have the essential immunological and biological properties as referred to above. Preferably, the cardiac troponin variants have immunological properties (i.e. epitope composition) comparable to those of human troponin T or troponin I. Thus, the variants shall be recognizable by the aforementioned means or ligands used for determination of the concentration of the cardiac troponins. Thus, the variants shall be recognizable by the aforementioned means or ligands used for determination of the concentration of the cardiac troponins. Such fragments may be, e.g., degradation products of the Troponins. Further included are variants which differ due to posttranslational modifications such as phosphorylation or myristylation. Preferably the biological property of troponin I and its variant is the ability to inhibit actomyosin ATPase or to inhibit angiogenesis in vivo and in vitro, which may e.g. be detected based on the assay described by Moses et al. 1999 PNAS USA 96 (6): 2645-2650). Preferably the biological property of troponin T and its variant is the ability to form a complex with troponin C and I, to bind calcium ions or to bind to tropomyosin, preferably if present as a complex of troponin C, I and T or a complex formed by troponin C, troponin I and a variant of troponin T. It is known that low concentrations of circulating cardiac troponin may be detected in subjects at various conditions, but further studies are required to understand their respective role and rate (Masson et al., Curr Heart Fail Rep (2010) 7:15-21).

The term "sample" refers to a sample of a body fluid, to a sample of separated cells or to a sample from a tissue or an organ. Samples of body fluids can be obtained by well known techniques and include, preferably, samples of blood, plasma, serum, urine, more preferably blood, plasma and serum. It is to be understood that the sample depends on the marker to be determined. Therefore, it is encompassed that the polypeptides as referred to herein are determined in different samples. A-FABP or a variant thereof, adiponectin or a variant thereof, RBP-4 or a variant thereof, and CRP or a variant thereof are, preferably, determined in a blood serum or blood plasma sample. Cardiac troponins or a variant thereof, are, preferably, determined in a blood serum or blood plasma sample.

Determining the amount of A-FABP or a variant thereof, adiponectin or a variant thereof, CRP or a variant thereof or any other peptide or polypeptide referred to in this specification relates to measuring the amount or concentration, preferably semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labelled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse-proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR-analyzers, or chromatography devices. Further, methods include micro-plate ELISA-based methods, fully-automated or robotic immunoassays (available for example on Elecsys™ analyzers), CBA (an enzymatic Cobalt Binding Assay, available for example on Roche-Hitachi™ analyzers), and latex agglutination assays (available for example on Roche-Hitachi™

analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (β) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprise the steps of (α) contacting the peptide with a specific ligand, (optionally) removing non-bound ligand, (β) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)$_2$ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable label prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand. Labelling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include $^{35}S$, $^{125}I$, $^{32}P$, $^{33}P$ and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other detection methods as described above.

The amount of a peptide or polypeptide may be, also preferably, determined as follows: (α) contacting a solid support comprising a ligand for the peptide or polypeptide as specified above with a sample comprising the peptide or polypeptide and (β) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived there from. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample or a ratio of amounts is compared to a reference ratio of amounts. The comparison referred to in step (c) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format.

Based on the comparison of the amount(s) determined in step a) to suitable reference amount(s), it is possible to predict the risk of a HIV infected individual to suffer from a cardiac complication and/or death. It is to be understood that amounts of A-FABP or a variant thereof and, as the case may be, adiponectin or a variant thereof and/or CRP or a variant thereof as determined in step (a) of the methods of the presents invention are compared in step (b) to reference amounts for of A-FABP or a variant thereof and, as the case may be, adiponectin or a variant thereof and/or CRP or a variant thereof as specified elsewhere in this application.

The term "reference amounts" as used herein in this embodiment of the invention refers to amounts of the polypeptides which allow diagnosing metabolic alterations in a HIV infected subject potentially leading to a cardiac complication or death and/or predicting the risk of a HIV infected individual of suffering from a cardiac complication and/or death (or, in other words, assessing or diagnosing if a HIV infected individual has an increased risk of suffering from a cardiac complication and/or death).

Therefore, the reference amounts will in general be derived from subjects known to be physiologically healthy, or HIV infected subjects, preferably those undergoing an antiretroviral therapy, in particular HAART, which subjects are known to suffer from metabolic alterations potentially leading to a cardiac complication or death (and wherein the subjects may be apparently healthy for diseases other than a HIV infection), or HIV infected subjects, preferably those undergoing an antiretroviral therapy, in particular HAART, which subjects are known to suffer or having suffered from metabolic alterations and which subjects have experienced a cardiac complication or death.

Accordingly, the term "reference amount" as used herein either refers to an amount which allows diagnosing metabolic alterations in a HIV infected subject potentially leading to a cardiac complication or death and/or predicting the risk of a HIV infected individual of suffering from a cardiac complication and/or death, and wherein the amounts of the respective marker(s) can be determined prior to the cardiac complication and/or death. The comparison with reference amounts permits diagnosing metabolic alterations in a HIV infected subject potentially leading to a cardiac complication or death and/or predicting the risk of a HIV infected individual of suffering from a cardiac complication and/or death. The individual's pathological state relative to a HIV infection, preferably, has been established prior to determining the markers of the present invention.

In all embodiments of the present invention, the amount/amounts of the respective markers used therein (A-FABP or a variant thereof and, as the case may be, adiponectin or a variant thereof and/or CRP or a variant thereof) are determined by methods known to the person skilled in the art.

In general, for determining the respective amount(s)/amount(s) or amount ratios allowing to establish the desired diagnosis in accordance with the respective embodiment of the present invention, ("threshold", "reference amount"), the amount(s)/amount(s) or amount ratios of the respective peptide or peptides are determined in appropriate patient groups. According to the diagnosis to be established, the patient group may, for example, comprise only healthy individuals, or may comprise healthy individuals and individuals suffering from the pathophysiological (state which is to be determined, or may comprise only individuals suffering from the pathophysiological state which is to be determined, or may comprise individuals suffering from the various pathophysiological states to be distinguished, by the respective marker(s) using validated analytical methods. The results which are obtained are collected and analyzed by statistical methods known to the person skilled in the art. The obtained threshold values are then established in accordance with the desired probability of suffering from the disease and linked to the particular threshold value. For example, it may be useful to choose the median value, the 60th, 70th, 80th, 90th, 95th or even the 99th percentile of the healthy and/or non-healthy patient collective, in order to establish the threshold value(s), reference value(s) or amount ratios.

A reference value of a diagnostic marker can be established, and the amount of the marker in a patient sample can simply be compared to the reference value. The sensitivity and specificity of a diagnostic and/or prognostic test depends on more than just the analytical "quality" of the test-they also depend on the definition of what constitutes an abnormal result. In practice, Receiver Operating Characteristic curves, or "ROC" curves, are typically calculated by plotting the value of a variable versus its relative frequency in "normal" and "disease" populations. For any particular marker of the invention, a distribution of marker amounts for subjects with and without a disease will likely overlap. Under such conditions, a test does not absolutely distinguish normal from disease with 100% accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition. ROC curves can be used even when test results don't necessarily give an accurate number. As long as one can rank results, one can create an ROC curve. For example, results of a test on "disease" samples might be ranked according to degree (say 1=low, 2=normal, and 3=high). This ranking can be correlated to results in the "normal" population, and a ROC curve created. These methods are well known in the art. See, e.g., Hanley et al, Radiology 143: 29-36 (1982).

In certain embodiments, markers and/or marker panels are selected to exhibit at least about 70% sensitivity, more preferably at least about 80% sensitivity, even more preferably at least about 85% sensitivity, still more preferably at least about 90% sensitivity, and most preferably at least about 95% sensitivity, combined with at least about 70% specificity, more preferably at least about 80% specificity, even more preferably at least about 85% specificity, still more preferably at least about 90% specificity, and most preferably at least about 95% specificity. In particularly preferred embodiments, both the sensitivity and specificity are at least about 75%, more preferably at least about 80%, even more preferably at least about 85%, still more preferably at least about 90%, and most preferably at least about 95%. The term "about" in this context refers to +/- 5% of a given measurement.

In other embodiments, a positive likelihood ratio, negative likelihood ratio, odds ratio, or hazard ratio is used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group. In certain preferred embodiments, markers and/or marker panels are preferably selected to exhibit a positive or negative likelihood ratio of at least about 1.5 or more or about 0.67 or less, more preferably at least about 2 or more or about 0.5 or less, still more preferably at least about 5 or more or about 0.2 or less, even more preferably at least about 10 or more or about 0.1 or less, and most preferably at least about 20 or more or about 0.05 or less. The term "about" in this context refers to +/- 5% of a given measurement.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In certain preferred embodiments, markers and/or marker panels are preferably selected to exhibit an odds ratio of at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less. The term "about" in this context refers to +/- 5% of a given measurement.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group. In certain preferred embodiments, markers and/or marker panels are preferably selected to exhibit a hazard ratio of at least about 1.1 or more or about 0.91 or less, more preferably at least about 1.25 or more or about 0.8 or less, still more preferably at least about 1.5 or more or about 0.67 or less, even more preferably at least about 2 or more or about 0.5 or less, and most preferably at least about 2.5 or more or about 0.4 or less. The term "about" in this context refers to +/- 5% of a given measurement.

While exemplary panels are described herein, one or more markers may be replaced, added, or subtracted from these exemplary panels while still providing clinically useful results. Panels may comprise both specific markers of a disease (e.g., markers that are increased or decreased in bacterial infection, but not in other disease states) and/or non-specific markers (e.g., markers that are increased or decreased due to inflammation, regardless of the cause; markers that are increased or decreased due to changes in hemostasis, regardless of the cause, etc.). While certain markers may not individually be definitive in the methods described herein, a particular "fingerprint" pattern of changes may, in effect, act as a specific indicator of disease state. As discussed above, that pattern of changes may be obtained from a single sample, or may optionally consider temporal changes in one or more members of the panel (or temporal changes in a panel response value).

In order to test if a chosen reference value yields a sufficiently safe diagnosis of patients suffering from the disease of interest, one may for example determine the efficiency (E) of the methods of the invention for a given reference value using the following formula:

$$E = (TP / TO) \times 100;$$

wherein TP = true positives and TO = total number of tests = TP + FP + FN + TN, wherein FP = false positives; FN = false negatives and TN = true negatives. E has the following range of values: 0 < E < 100). Preferably, a tested reference value yields a sufficiently safe diagnosis provided the value of E is at least about 50, more preferably at least about 60, more preferably at least about 70, more preferably at least about 80, more preferably at least about 90, more preferably at least about 95, more preferably at least about 98.

The diagnosis if individuals are healthy or suffer from a certain pathophysiological state is made by established methods known to the person skilled in the art. The methods differ in respect to the individual pathophysiological state.

The algorithms to establish the desired diagnosis are laid out in the present application, in the passages referring to the respective embodiment, to which reference is made.

Accordingly, the present invention also comprises a method of determining the threshold amount indicative for a physiological and/or a pathological state and/or a certain pathological state, comprising the steps of determining in appropriate patient groups the amounts of the appropriate marker(s), collecting the data and analyzing the data by statistical methods and establishing the threshold values. Here, the physiological and/or pathological state is a metabolic alteration, preferably a lipid and/or glucose metabolic alteration, in a HIV infected subject which preferably undergoes an antiretroviral treatment, in particular HAART, and wherein the metabolic alteration is known to potentially lead to a cardiac complication and/or death, or wherein the metabolic alteration has lead to a cardiac complication and/or death in the individual.

The term "about" as used herein refers to +/- 20%, preferably +/-10%, preferably, +/- 5% of a given measurement or value.

The terms "low risk", "lower risk", "very low risk", "high risk", "high risk", " very high risk" used in this context, i.e. are known to the person skilled in the art. The person skilled in the art is able to determine actual values for the relevant biochemical markers which correspond to these levels.

For example, the levels may be assigned according to percentiles of the levels observed in a representative sample of apparently healthy individuals below an age of 50 years (preferably, the sample comprises at least 100, more preferably at least 500, most preferably at least 1000 individuals). E.g., a non-increased level may correspond to the maximum level observed in the 97.5% percentile.

Alternatively, the levels may be determined as "normal ranges" as known in the state of the art. The levels may also be determined or further refined by studies performed on individuals undergoing stress testing and correlating any adverse events with the levels observed in the individuals. Such studies may also allow to tailor the levels according to certain patient subgroups, e.g. patients with known coronary artery disease, elderly patients, or apparently healthy individuals. Guidance on how such studies may be carried out can also be obtained from the Examples included in this application.

The value of the levels considered as corresponding to a "low risk", "lower risk", "very low risk", "high risk", "high risk", "very high risk" may also be chosen according to the desired sensitivity or specificity (stringency) of exclusion. The higher the desired sensitivity, the lower is the specificity of exclusion and vice versa. In the above example, the higher the percentile chosen to determine each level, the more stringent is the exclusion criterion, i.e. less individuals would be considered "risk individuals".

Below, examples for actual levels are provided for all the markers cited in the context of the present invention. It is evident, that the levels given below can serve only as a first classification of the risk of an individual. For example, the risk may also dependent on an existing metabolic syndrome of a particular individual or on the general health status of the individual.

In a preferred embodiment of the present invention, the following reference amounts for the markers are used. Average risk depends among others on the underlying disease, comorbidities and type of medication of the HIV infected subject. The terms "high risk", "higher risk", "very high risk" as used in the context of the present invention relate to those cases where the risk is increased relative to average risk. The terms "low risk", "lower risk", "very low risk" as used in the context of the present invention relate to those cases where the risk is decreased relative to average risk.

A-FABP:

A reference amount of ≤ about 12 ng/ml, preferably ≤ about 10 ng/ml, more preferably ≤ about 8 ng/ml, A-FABP or a variant thereof, is indicative that the individual is at a low risk, preferably at lower risk, more preferably at a very low risk of suffering from a cardiac complication and/or death (rule out).

A reference amount of ≥ about 14 ng/ml, preferably ≤ about 16 ng/ml, more preferably ≥ about 18 ng/ml, in particularly ≥ about 20 ng/ml A-FABP or a variant thereof, is indicative that the individual is at a high risk, preferably at higher risk, more preferably at a very high risk of suffering from a cardiac complication or death (rule in).

Adiponectin:

In case additionally the amount of adiponectin or a variant thereof is determined, the reference values or reference amounts indicating a risk of suffering from a cardiac complication or death are the following:

A reference amount of ≥ about 1.8 μg/ml, preferably ≥ about 2.2 μg/ml, more preferably ≥ about 2.5 μg/ml, in particular ≥ about 3.0 μg/ml adiponectin or a variant thereof, is indicative that the individual is at a low risk, preferably at lower risk, more preferably at a very low risk of suffering from a cardiac complication and/or death (rule out).

A reference amount of < about 1.8 μg/ml, preferably ≤ about 1.5 μg/ml, more preferably ≤ about 1.0 μg/ml, adiponectin or a variant thereof, is indicative that the individual is at a high risk, preferably at higher risk, more preferably at a very high risk of suffering from a cardiac complication and/or death (rule in).

Adiponectin gives information independent from A-FABP

hs CRP

In case additionally the amount of CRP or a variant thereof is determined, the reference values indicating a risk of suffering from a cardiac complication or death are the following:

A reference amount of ≤ about 1.8 mg/L, preferably ≤ about 1.3 mg/L, more preferably ≤ about 0.9 mg/L, CRP or a variant thereof, is indicative that the individual is at a low risk, preferably at lower risk, more preferably at a very low risk of suffering from a cardiac complication and/or death (rule out).

A reference amount of > about 1.8 mg/L, preferably ≥ about 2.5 mg/L, more preferably ≥ about 3.6 mg/L, CRP or a variant thereof, is indicative that the individual is at a high risk, preferably at higher risk, more preferably at a very high risk of suffering from a cardiac complication and/or death (rule in).

**Cardiac Troponins:**

It is known to the person skilled in the art that elevated levels of a cardiac troponin indicate an increased cardiovascular risk, see Omland, Journal of Internal Medicine 268 (2010), pages 207-217. It is generally recognized and known to the person skilled in the art that troponin levels higher than 3 pg/ml indicate an increased risk for a cardiac complication, in particular heart failure and/or death.

Determining the amount of a cardiac troponin can be used for confirming the diagnosis or prognosis or prediction established by the comparison of the markers used in accordance with the present invention, on the one hand. On the other hand, it permits to establish if the metabolic alteration as evidenced by the elevated marker(s) used in accordance with the present invention has already affected the cardiac system or not.

The person skilled in the art is aware that the concentrations cited in the present application for the cardiac troponins (troponin T or a variant thereof and troponin I or a variant thereof), may not apply for patients suffering from impaired renal function, preferably patients suffering from renal failure, in particular patients suffering from chronic and end stage renal failure. In a preferred embodiment of the present invention, patients suffering from impaired renal function, preferably patients suffering from renal failure, in particular patients suffering from chronic and end stage renal failure are not comprised in (excluded from) the methods of the present invention. In another preferred embodiment, patients with renal hypertension are not comprised in (excluded from) the methods of the present invention. Preferably, the "subject" as used herein excludes patients suffering from impaired renal function, preferably patients suffering from renal failure, in particular patients suffering from chronic and end stage renal failure, more preferably patients with renal hypertension, most preferably all of the patients suffering from one of the diseases and conditions mentioned in this sentence. In this context, "renal failure" is regarded as an impaired glomerular filtration rate (GFR) lying below the usual ranges of 60 to 120 ml/min, preferably below 60 ml/min. Chronic renal failure is a long-standing, progressive deterioration of renal function which often results in end stage renal failure. End stage renal failure is diagnosed when the GFR reaches a rate of up to about 30 ml/min. GFR is determined by the creatinine clearance, which is known to the person skilled in the art. Subjects with impaired renal function show higher levels of troponin I and troponin T than those cited above, due to an impaired clearance of the peptide. The levels vary with the severity of the renal impairment.

The severity of renal impairment is divided into various grades, as displayed below.

0:   ≥ 90 ml/min

1:   ≥ 90 ml/min with microalbuminuria

2:   ≥ 60 - < 90 ml/min

3:   ≥ 30 - < 60 ml/min

4:   ≥ 15 - < 30 ml/min

5:   < 15 ml/min

(Source: National Kidney Foundation, as published in: Am J. Kidney Dis 39 suppl 1, 2002; Clinical Practice Guidelines for chronic kidney disease).

In a further aspect, the present invention provides a method of monitoring the treatment of a HIV infected subject, preferably based on the repeated determination of the amount of A-FABP in a sample of the subject and comparing the amount to reference amounts.

The method comprises at least one of the following steps: a) repeatedly determining the amount of A-FABP or a variant thereof in a sample of the subject; b) comparing the repeatedly determined amounts to at least one reference amount; c) monitoring the therapy, based on the comparison carried out in step b).

Thus, the present invention also provides a method of monitoring the treatment of a HIV infected subject, comprising the steps of

a) determining the amount of A-FABP or a variant thereof in a sample of the subject after therapy initiation at least once, or repeatedly within a given time interval;
b) comparing the repeatedly determined amounts to at least one reference amount;
c) monitoring the treatment, based on the comparison carried out in step b).

Preferably, monitoring includes a step bi) in which the risk of the subject of suffering from an adverse cardiac complication and/or death is determined, by comparing the repeatedly determined amounts to reference amounts. The monitoring step then includes the information relative to the risk implied in accordance with the repeatedly determined amounts.

In accordance with the foregoing, monitoring according to the present invention is based on the repeated determination of the risk of the HIV infected individual to suffer from a cardiac complication and/or death.

In a preferred embodiment of the present invention, the amount of adiponectin or a variant thereof is determined in the sample, further to the amount of A-FABP or a variant thereof.

In further preferred embodiment of the present invention, the amount of C-reactive protein CRP or a variant thereof is determined in the sample, further to the amount of A-FABP or a variant thereof and, optionally, adiponectin or a variant thereof.

Preferably and in general, the HIV infected subject is under an antiretroviral therapy, preferably HAART.

Thus, "monitoring" as used herein relates to keeping track of the pathophysiological state of the respective individual relative to the risk of suffering from a cardiac complication or death, occurrence and/or progression of the disease or the influence of a particular treatment on the progression of disease. "Monitoring" as used herein relates to keeping track of the pathophysiological state of the respective individual relative to metabolic alterations, need for adaption of antiretroviral therapy and/or adaptation of treatment preventing cardiac complications or death, occurrence and/or progression of the disease or the influence of a particular treatment on the progression of disease.

In the present invention, the sample is obtained at an appropriate time-point after initiation of the therapy, which is known by the skilled person. It has to be taken into consideration that the subject under examination does not suffer from an acute pathophysiological state, accordingly not requiring rapid diagnosis and a rapid decision on an appropriate treatment. Preferably, the sample is obtained from a subject according to the present invention within e.g. about 1 month, preferably not more than within about 2 months, within not more than about 3 months, within not more than about 4 months, within not more than about 5 months, within not more than about 6 months, or within not more than about 12 months after beginning antiretroviral therapy, preferably HAART. Preferably, the sample will be taken within about 3 to 6 months) after beginning antiretroviral therapy, preferably HAART.

In case the amounts of A-FABP or a variant thereof and, optionally, the amount of adiponectin or a variant thereof and/or the amount of C-reactive protein CRP or a variant thereof are determined in the sample, are determined repeatedly, preferably at least twice, to monitor the treatment of a HIV infected subject, the second and each further sample will be taken in an interval which ensures an effective monitoring of the metabolic state and/or its alterations. In general, the interval between each sample is about 2 months, about 3 months, about 4 months, about 5 months, about 6 months, preferably between about 3 to 6 months after the previous sample has been taken. For example, the initial samples is taken about 3 to 6 months after beginning antiretroviral therapy, preferably HAART, and each further sample is taken about 3 to 6 months after the previous samples. The number of samples taken will depend on the evaluation of the HIV treatment. It may be sufficient to take one sample and determine the amount of the respective marker(s), e.g. in case the amount(s) of the marker(s) show a significant elevation relative to normal values. Frequently, the marker(s) will be determined several times, i.e. 2, 3, 4, 5, 6, 7, 8, 9, 10, 15, 20 or 30 times. In this aspect of the present invention, a amount equal to or higher than the reference value for the respective marker (see above) is indicative of an elevated risk of a cardiac complication and/or death and/or of a metabolic alteration potentially leading to a cardiac complication and/or death.

In a further aspect, the present invention provides a method of recommending or deciding on the adaptation of the treatment of a HIV infected subject, preferably based on the repeated determination of the amount of A-FABP in a sample of the subject and comparing the amount to reference amounts.

The method comprises at least one of the following steps: a) repeatedly determining the amount of A-FABP or a variant thereof in a sample of the subject; b) comparing the repeatedly determined amounts to at least one reference amount; c) monitoring the therapy, based on the comparison carried out in step b).

Thus, the present invention also provides a method of recommending or deciding on the adaptation of the treatment of a HIV infected subject, comprising the steps of

a) determining the amount of A-FABP or a variant thereof in a sample of the subject after therapy initiation at least once, or repeatedly within a given time interval;

b) comparing the repeatedly determined amounts to at least one reference amount;

c) recommending or deciding on the treatment, based on the comparison carried out in step b).

Preferably, monitoring includes a step bi) in which the risk of the subject of suffering from an adverse cardiac complication and/or death is determined, by comparing the repeatedly determined amounts to reference amounts. Also preferably, the method comprises a step bii) wherein the treatment is monitored, and wherein monitoring is carried out based on the information obtained in step b), and/or the information obtained in step bi), preferably the information obtained in steps b) and bi). The step of recommending and/or deciding then includes the information obtained in step b), and/or the information obtained in step bi), and/or the information obtained in step bii), preferably the information obtained in steps b), bi) and bii) based on information in connection with the repeatedly determined amounts.

In a preferred embodiment of the present invention, the amount of adiponectin or a variant thereof is determined in the sample, further to the amount of A-FABP or a variant thereof.

In a further preferred embodiment of the present invention, the amount of C-reactive protein CRP or a variant thereof is determined in the sample, further to the amount of A-FABP or a variant thereof and, optionally, adiponectin or a variant thereof.

Preferably and in general, the HIV infected subject is under an antiretroviral therapy, preferably HAART.

In this embodiment of the present invention, the method of monitoring allows to assess if a HIV infected individual who receives an antiretroviral therapy, preferably HAART, is developing a risk of suffering from a cardiac complication or death, or if an existing risk in the HIV infected individual of suffering from a cardiac complication or death has increased or lowered or remained constant, as the case may be. In this embodiment, the same reference amounts/reference values which are disclosed beforehand apply.

The risk is assessed based on the determined amounts of A-FABP and, optionally, the amount(s) of adiponectin, and/or CRP determined in a sample of the subject.

If in the method of monitoring it is assessed that a risk of suffering from a cardiac complication or death is about to develop, or that an existing risk in the HIV infected individual of suffering from a cardiac complication or death has increased, it will in general be recommended to adapt the antiretroviral treatment/HAART, and/or it will be decided to adapt the antiretroviral treatment/HAART.

Adaption can be achieved by changing the antiretroviral treatment, e.g. by administering a different class of antiretroviral drugs (i.e. changing the drug within the classes of nRTIs, NRTIs, NNRTIs, PIs, EIs (fusion inhibitors), and integrase inhibitors). It is also possible to modify the combination of the antiretroviral drugs.

If the subject shows clear metabolic alterations potentially those leading to a cardiac complication, the antiretroviral therapy/HAART will be adapted to those antiretroviral drugs/antiretroviral drug classes which do not trigger or only trigger to a lesser amount the occurrence of metabolic alterations potentially leading to a cardiac complication. These have been specified beforehand and include the following:

PIs, NNRTIs + nRTIs ; NNRTIs + NRTIs + nRTIs; NRTIs ; NRTIs + nRTIs (I; II + III + IV ; III ; III + IV)

The roman numbers have the following meaning : Nucleotide reverse transcriptase inhibitors (nRTIs) IV; Nucleoside reverse transcriptase inhibitors (NRTIs) III; Non-nucleoside reverse transcriptase inhibitors (NNRTIs) II; and Protease inhibitors (PIs) I.

In a further embodiment, it is possible, to administer medicaments which lower the risk of suffering from a cardiac complication/death. This can be carried out with or without adapting the antiretroviral therapy/HAART.

These medicaments are known to the person skilled in the art and include the following:

ACE inhibitors, in particular Enalapril, Captopril, Ramipril, Trandolapril; angiotensin receptor antagonists and aldosterone antagonists, in particular Losartan, Valsartan, Irbesartan, Candesartan, Telmisartan, Eprosartan, Spironolactone; statines, in particular Atorvastatin, Fluvastatin, Lovastatin, Pravastatin, Rosuvastatin, Simvastatin; beta blockers like proprenolol, metoprolol, bisoprolol, carvedilol, bucindolol, nebivolol; nitrates; adrenergic agonists, like dobutamine, dopamine, epinephrine, isoprotenerol, norepinephrine, phenylephrine; antiplatelet agents, in particular aspirin and clopidrogel; anticoagulants, in particular warfarin, heparin, thrombin inhibitors, thrombinolytic drugs; diuretics like loop diuretics, thiazide and thiazide-like diuretics, K-sparing diuretics, type I mineralocorticoid receptor antagonists, antialdosterone, carbonic anhydrase inhibitors, vasopressure antagonists, thiazolidine diones.

In the above-cited methods of monitoring and deciding on a therapy, the subject, preferably and in most cases, is a HIV-infected subject to whom an antiretroviral therapy is administered. The antiretroviral therapy is preferably a highly active antiretroviral therapy HAART.

In a preferred embodiment of the above-cited methods of recommending/deciding on and monitoring of a therapy of the present invention, the amount of adiponectin or a variant thereof is determined in the sample, further to the amount of A-FABP or a variant thereof, and the risk is predicted based on the comparison of the marker amounts with reference amounts

In further preferred embodiment of the above-cited methods of recommending/deciding on and monitoring of a therapy of the present invention, the amount of C-reactive protein CRP or a variant thereof is measured in the sample, further to the amount of A-FABP or a variant thereof and, optionally, adiponectin or a variant thereof, and the metabolic alterations are diagnosed and/or the risk is predicted based on the comparison of the marker amounts with reference amounts.

In a further embodiment, the amount of a cardiac troponin, preferably troponin T or troponin I, is determined.

The present invention also provides a method of assessing the metabolic effect and/or the potential to cause a cardiac complication, of an antiretroviral drug, comprising the steps of

a) determining the amount of A-FABP or a variant thereof in a sample of the subject
b) comparing the amount to reference amount
c) diagnosing metabolic alterations in the subject, based on the comparison carried out in step b);
d) assessing the metabolic effect of the antiretroviral drug.

The above method can be used for screening several antiviral drugs on their respective metabolic effect. "Several" means at least 2, or 3, 4, 5, 6, 7, 8, 9, 10.......

In a preferred embodiment of the present invention, the amount of adiponectin or a variant thereof is determined in the sample, further to the amount of A-FABP or a variant thereof.

In a further preferred embodiment of the present invention, the amount of C-reactive protein CRP or a variant thereof is determined in the sample, further to the amount of A-FABP or a variant thereof and, optionally, adiponectin or a variant thereof.

Preferably and in general, the HIV infected subject is under an antiretroviral therapy, preferably HAART.

The above method of assessing the metabolic effect and/or the potential to cause a cardiac complication of an antiretroviral drug is also a method for screening an antiretroviral drug on its metabolic effect and/or its potential to cause a cardiac complication.

In one embodiment, the present invention encompasses a method for diagnosing myocardial infarction in a subject, comprising the steps of:

a) determining the amounts of a cardiac troponin or a variant thereof, in a subject;
b) comparing the amounts determined in step a) with reference amounts; and
c) diagnosing myocardial infarction based on the steps carried out in step b).

Preferably, the cardiac troponin is troponin T or troponin I.

In a further embodiment, the present invention encompasses a method for diagnosing heart failure in a subject, comprising the steps of:

a) determining the amounts of a natriuretic peptide or a variant thereof, in a subject;
b) comparing the amounts determined in step a) with reference amounts; and
c) diagnosing heart failure based on the steps carried out in step b).

Preferably, the natriuretic peptide is BNP, NT-proBNP, ANP, or NT-proANP, preferably NT-proBNP.

Moreover, the present invention also envisages kits and devices adapted to carry out the method of the present invention.

Furthermore, the present invention provides a device for diagnosing metabolic alterations potentially leading to a cardiac complication or death and/or predicting the risk to suffer from a cardiac complication or death, in a HIV infected subject, comprising:

a) an analyzing unit for determining the amounts of A-FABP or a variant thereof, and, as the case may be, adiponectin or a variant thereof and/or RBP-4 or a variant thereof and/or CRP or a variant thereof, in a sample of a subject;
b) an evaluation unit for comparing the amounts determined in step a) with reference amounts.

Preferably, the kit is adapted for diagnosing metabolic alterations and/or predicting the risk.

Moreover the present invention provides a kit for diagnosing metabolic alterations potentially leading to a cardiac complication or death and/or predicting the risk to suffer from a cardiac complication or death, in a HIV infected subject, comprising:

a) a specific ligand for determining the amounts of A-FABP or a variant thereof, and, as the case may be, adiponectin or a variant thereof and/or RBP-4 or a variant thereof and/or CRP or a variant thereof, in a sample of a subject;
b) an evaluation unit for comparing the amounts determined in step a) with reference amounts.

Preferably, the kit is adapted for diagnosing metabolic alterations and/or predicting the risk.

The term "device" as used herein relates to a system of means comprising at least the aforementioned means operatively linked to each other as to allow, in a HIV infected subject, for diagnosing metabolic alterations potentially leading to a cardiac complication or death and/or predicting the risk to suffer from a cardiac complication or death, and/or monitoring the treatment of a HIV infected subject, and/or recommending or deciding on the adaptation of the treatment of a HIV infected subject. Preferred means for determining the amount of A-FABP, and, as the case may be, adiponectin or a variant thereof and/or RBP-4 or a variant thereof and/or CRP or a variant thereof, and means for carrying out the comparison are disclosed above in connection with the method of the invention. How to link the means in an operating manner will depend on the type of means included into the device. For example, where an analysis unit for automatically determining the amount of the peptides are applied, the data obtained by said automatically operating means can be processed by, e.g., a computer program in order to obtain the desired results.

Said device, preferably, includes an analyzing unit for the measurement of the amount of A-FABP and/or adiponectin and/or CRP in an applied sample and an evaluation unit for processing the resulting data. Preferably, the evaluation unit comprises a database with the stored reference amounts and a computer program code which when tangibly embedded on a computer carries out the comparison of the determined amounts and the reference amounts stored in the database. More preferably, the evaluation unit comprises a further computer program code which allocates the result of the comparison to a risk prediction. In such a case, it is, also preferably, envisaged that the evaluation unit comprises a further database wherein the reference amounts are allocated to the risks.

Alternatively, where means such as test stripes are used in the analyzing unit for determining the amount of A-FABP and/or adiponectin and/or CRP, the evaluation unit may comprise control stripes or tables allocating the determined amount to a reference amount. The test stripes are, preferably, coupled to a ligand which specifically binds to A-FABP and/or adiponectin and/or CRP. The strip or device, preferably, comprises means for detection of the binding of said A-FABP and/or adiponectin and/or CRP to the said ligand. Preferred means for detection are disclosed in connection with embodiments relating to the method of the invention above. In such a case, the analysis unit and the evaluation unit are operatively linked in that the user of the system brings together the result of the determination of the amount and the diagnostic or prognostic value thereof due to the instructions and interpretations given in a manual. The analysis unit and the evaluation unit may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of raw data which need interpretation by the clinician. Preferably, the output of the device is, however, processed, i.e. evaluated, raw data the interpretation of which does not require a clinician. Further preferred devices comprise the analyzing units/devices (e.g., biosensors, arrays, solid supports coupled to ligands specifically recognizing the gene product, Plasmon surface resonance devices, NMR spectrometers, mass-spectrometers etc.) or evaluation units/devices referred to above in accordance with the method of the invention.

In a preferred embodiment of the present invention the analyzing unit of the device further comprises means for determining the amounts of at least one marker selected from the group consisting of A-FABP and/or adiponectin and/or CRP in a sample of a subject and the evaluation unit comprises means for comparing said amounts to reference amounts.

The term "kit" as used herein refers to a collection of the aforementioned components of which may or may not be packaged together. The components of the kit may be comprised by separate vials (i.e. as a kit of separate parts) or provided in a single vial. Moreover, it is to be understood that the kit of the present invention is to be used for practising the methods referred to herein above. It is, preferably, envisaged that all components are provided in a ready-to-use manner for practising the methods referred to above. Further, the kit preferably contains instructions for carrying out the said methods. The instructions can be provided by a user's manual in paper- or electronic form. For example, the manual may comprise instructions for interpreting the results obtained when carrying out the aforementioned methods using the kit of the present invention. The kit shall comprise a specific ligand. This specific ligand is capable of specifically recognizing A-FABP and/or adiponectin and/or CRP in a sample of the subject. Moreover, said specific ligand shall upon binding to the A-FABP and/or adiponectin and/or CRP , preferably, be capable of generating a detectable signal, the intensity of which correlates to the amount of A-FABP and/or adiponectin and/or CRP present in the sample. Dependent on the type of signal which is generated, methods for detection of the signal can be applied which are well known in the art. Specific ligands which are preferably used for the kit of the present invention include antibodies or aptamers.The specific ligand may be present on a test stripe as described elsewhere herein. The amounts of A-FABP and/or adiponectin and/or CRP thus detected can then be further evaluated in the evaluation unit. Preferred evaluation units to be used for the kit of the present invention include those referred to elsewhere herein.

In a preferred embodiment of the present invention the kit further comprises a specific ligand/specific ligands for determining the amount of at least one marker selected from the group consisting of markers A-FABP and/or adiponectin and/or CRP in a sample of a subject and an evaluation unit for comparing said amount ratios to reference amounts.

The present invention also relates to the use of a kit or device for determining the amount of A-FABP or a variant thereof, and, as the case may be, adiponectin or a variant thereof and/or CRP or a variant thereof, in a sample of a subject; and/or the use of means for determining the amount of A-FABP or a variant thereof, and, as the case may be, adiponectin or a variant thereof and/or CRP or a variant thereof; and/or the use of means for comparing the amount of A-FABP or a variant thereof, and, as the case may be, adiponectin or a variant thereof and/or CRP or a variant thereof, to at least one reference amount, for: diagnosing metabolic alterations potentially leading to a cardiac complication or death and/or predicting the risk to suffer from a cardiac complication or death, and/or monitoring the treatment of a HIV infected subject, and/or recommending or deciding on the adaptation of the treatment of a HIV infected subject.

The present invention also relates to the use of: an antibody to A-FABP or a variant thereof, and, as the case may be, an antibody to adiponectin or a variant thereof and/or an antibody to CRP or a variant thereof; and/or of means for determining the amount of A-FABP or a variant thereof, and, as the case may be, means for determining the amount of adiponectin or a variant thereof and/or means for determining the amount of CRP or a variant thereof; and/or of means for comparing the amount of A-FABP or a variant thereof, and, as the case may be, adiponectin or a variant thereof and/or CRP or a variant thereof, to at least one reference amount, for the manufacture of a diagnostic composition for: diagnosing metabolic alterations potentially leading to a cardiac complication or death and/or predicting the risk to suffer from a cardiac complication or death, and/or monitoring the treatment of a HIV infected subject, and/or recommending or deciding on the adaptation of the treatment of a HIV infected subject.

The present invention also relates to the use of: an antibody to A-FABP or a variant thereof, and, as the case may be, an antibody to adiponectin or a variant thereof and/or an antibody to CRP or a variant thereof; and/or of means for determining the amount of A-FABP or a variant thereof, and, as the case may be, means for determining the amount of adiponectin or a variant thereof, and/or means for determining the amount of CRP or a variant thereof; and/or of means for comparing the amount of A-FABP or a variant thereof, and, as the case may be, adiponectin or a variant thereof and/or CRP or a variant thereof, to at least one reference amount for: diagnosing metabolic alterations potentially leading to a cardiac complication or death and/or predicting the risk to suffer from a cardiac complication or death, and/or monitoring the treatment of a HIV infected subject, and/or recommending or deciding on the adaptation of the treatment of a HIV infected subject.

All references cited in this specification are herewith incorporated by reference with respect to their entire disclosure content and the disclosure content specifically mentioned in this specification.

The following examples shall merely illustrate the invention. They shall not be construed, whatsoever, to limit the scope of the invention.

### Experimental section

### Determination of peptides

A-FABP was determined by the Biovendor HUMAN ADIPOCYTE FABP ELISA (HUMAN FABP4 ELISA from BioVendor - Laboratorni medicina a.s., CTPark Modrice Evropska 873 664 42 Modrice, Czech Republic. Samples are incubated in microplate wells pre-coated with polyclonal anti-human A-FABP antibody. After incubation and washing, biotin-labelled polyclonal anti-human A-FABP antibody is added and incubated with captured A-FABP. After another washing, streptavidin-HRP conjugate is added. After incubation and the last washing step, the remaining conjugate is allowed to react with the substrate solution (TMB). The reaction is stopped by addition of acidic solution and absorbance of the resulting yellow product is measured spectrophotometrically at 450 nm. The absorbance is proportional to the concentration of AFABP. The antibodies in the kit are highly specific for human AFABP with no detectable crossreactivities to human leptin, leptin receptor, adiponectin, resistin, HFABP, LFABP, IFABP, EFABP and RELM-beta at 50 ng/ml and IL-6, AGRP, ASP (C3adesArg) at 2 ng/ml. The A-FABP assay allows a measurement of A-FABP levels in the range of 0.1 to 25 ng/ml.

Adiponectin was determined by the Alpco Adiponectin (Multimeric) EIA (ELISA) from Alpco diagnostics, 26-G Keewaydin Drive, Salem, NH 03079. The kit utilizes a method of measurement incorporating pretreatment with proteases for selective measurement of human multimeric adiponectin. In addition, a simple pretreatment method is utilized. By the proposed method, multimeric adiponectin in the serum is converted mainly to a dimer via the addition of an SDS-containing acid buffer (without boiling step). The specific antibodies used in the kit are anti-human adiponectin monoclonal antibodies directed to two independent epitopes. The specimens are pre-treated as described below, and total adiponectin and individual multimers of adiponectin are determined selectively, directly or indirectly. The microtiter plates are coated with an anti-human adiponectin monoclonal antibody. Adiponectin in the standards and pretreated specimens are captured by the antibody during the first incubation. Afterwards, a wash step removes all unbound material. Subsequently, an anti-human adiponectin antibody which has been biotin-labeled is added and binds to the immobilized adiponectin in the wells. After the second incubation and subsequent wash step, HRP-labeled streptavidin is added. After the third incubation and subsequent wash step, substrate solution is added. The adiponectin assay allows a measurement of adiponectin levels in the range of 0.075 to 4.8 ng/ml.

CRP was determined by the Tina-quant cardiac CRP (Latex) High Sensitive test assay from Roche Diagnostics, Germany. The test contains a reagent solution with TRIS buffer and preservative to which the sample is added. Then a further reagent solution is added containing (together with buffer and preservative) an anti-CRP mouse monoclonal antibody which is pre-coated onto latex particles. The antibody/antigen complex agglutinates, and the amount is determined by turbidimetry. The CRP assay allows a measurement of CRP levels in the range of 0.1 to 20 mg/L.

Troponin T was determined using Roche's electrochemiluminescence ELISA sandwich test Elecsys Troponin T hs (high sensitive) STAT (Short Turn Around Time) assay. The test employs two monoclonal antibodies specifically directed against human cardiac troponin T. The antibodies recognize two epitopes (amino acid position 125-131 and 136-147) located in the central part of the cardiac troponin T protein, which consists of 288 amino acids. The hs-TnT assay allows a measurement of troponin T levels in the range of 1 to 10000 pg/mL.

**Example 1**

**Peptide levels in healthy/metabolic syndrome/type 2 diabetes/HIV infected subjects**

Subj ects/Patients:

A total of 149 healthy subjects (51 males , mean age 40 years, range 20 - 52 years, 98 females, mean age 41 years, range 18 to 56 years) served as a healthy control population. All subjects had normal blood pressure, a normal electrocardiogram, no diabetes mellitus or history of cardiac disease of any type and no cardiovascular risk factors such as smoking. In addition they had normal kidney function, i.e. a glomerular filtration rate higher than 60 ml per minute and 1.73 m$^2$ body surface area or by serum creatinine value lower than 1.3 mg/dl.

A total of 420 individuals (261 females, 159 males, mean age 48 years) meeting the criteria of the metabolic syndrome were included into the study. Metabolic syndrome was diagnosed according to the EGIR criteria (European Group for the Study of Insulin resistance).. It requires for the diagnosis/presence of the metabolic syndrome, insulin resistance defined as the top 25 % of the fasting insulin values among non-diabetic individuals, and two or more of the following:

a) central obesity: waist circumference above 94 cm (male), above 80 cm ( female) and(or body mass index (BMI) above 30 kg/m2
b) plasma triglycerides above 150 mg/dl and/or HDL-C above 39 mg/dl (or treated for dyslipemia)
c) hypertension: blood pressure above 140/90 mmHg (pr antihypertensive medication)
d) fasting plasma glucose above 6.1 mmol/l.

All individuals had normal kidney function and no history of cardiac disease.

A total of 52 patients with type 2 diabetes mellitus were included into the study. They had normal kidney function and no history of coronary artery disease. They were 41 males and 11 females, mean age 58 years.

In addition 300 HIV infected individuals were tested, all but 20 patients were on ART (active antiretroviral therapy) with different drug combinations. They were 280 males and 20 females, mean age 48 years (26 - 79 years). The majority of HIV infected individuals had HIV-RNA levels below the level of detectability of the test. Median CD 4 counts was 509/ul (range 252 - 1023) and thus not at increased risk of opportunistic infections. Antiviral therapy consisted of various combinations of antivirals of the following classes: Nucleotide reverse transcriptase inhibitors (nRTIs); Nucleoside reverse transcriptase inhibitors (NRTIs); Non-nucleoside reverse transcriptase inhibitors (NNRTIs); and Protease inhibitors (PIs).

Protease inhibitors (PIs) examples include Crixivan® (Indinavir), Kaletra® (Lopinavir and Ritonavir), Reyataz® (Atzanavir), Telzir® (Fosanprenavir), Invirase® (Saquinavir), Prezistza® (Duranavir). These antiretroviral drugs will be refered to in the present examples 1 and 2 as category I.

Non-nucleoside reverse transcriptase inhibitors (NNRTIs) examples include Sustiva® (Efavirenz), Emtriva® (Emtricabine). These antiretroviral drugs will be referred to in the present examples 1 and 2 as category II.

Nucleoside reverse transcriptase inhibitors (NRTIs) examples include Viramune® (Neviparin), Kivexa® (Abacavir and Lamivudine), Combivir® (Idovudine and Lamivudine), Retrovir® (Zidovudine), Videx® (DDI), Epivir® (Lamivudine), Ziagenavir® (Abacavir). These antiretroviral drugs will be referred to in the present examples 1 and 2 as category III.

Nucleotide reverse transcriptase inhibitors (nRTIs) examples include Truvada® (Teneofovir and Emtricabine) and Viread® (Tenofovir). These antiretroviral drugs will be referred to in the present examples 1 and 2 as category IV.

As depicted in Table 1, the following data were obtained in the four above-referenced groups of patients, results are given as median and the 25 und 75 percentile in parenthesis.

Table 1:

|  | Adiponectin μg/ml | A-FABP ng/ml | CRP mg/L | Troponin T * pg/ml |
|---|---|---|---|---|
| Healthy subject | 2.7 (1.6 - 4.0) | 13.7 (10.2 - 18.1) | 0.8 (0.4 - 1.8) | 0.0 (0.0 - 0.0) |
| Metabolic syndrome | 2.4 (1.5 - 3.5) | 23.2 (18.2 - 29.6) | 2.8 (1.5 - 5.3) | 3.7 (1.0 - 7.3) |
| Diabetes mellitus | 0.9 (0.7 - 1.6) | 24.6 (16.5 - 34.0) | 2.3 (1.1 - 5.4) | 10.7 (5.8 - 17.0) |
| HIV patients | 1.8 (1.0 - 2.9) | 12.1 (9.6 - 16.2) | 1.8 (0.9 - 3.6) | 0.0 (0.0 - 2.0) |
| * 0.0 refers to values below cut-off (1.0 pg/ml) | | | | |

Table 1

Table 1 shows that increased A-FABP concentrations are associated with long standing type 2 diabetes mellitus and the features of the metabolic syndrome, when compared to the healthy control group. It is also clear that these two disease categories do have increased troponin T levels which are associated with cardiac complications including heart failure, as indicated by troponin T levels. The troponin T levels are, however, well below the level indicative of acute cardiac events such as acute coronary syndrome. In contrast, HIV infected patients have similar A-FABP levels when compared to apparently health controls without diabetes mellitus and without signs of the metabolic syndrome (the HIV infected patients, however, differ from healthy subjects in that they have lower adiponectin levels which are even lower than in the metabolic syndrome but higher than in long standing type 2 diabetes mellitus). In addition, their troponin T levels are frequently undetectable with current sensitive tests indicating that HIV itself and average antiviral therapy are not associated with apparent cardiac disease.

The data show that patients with diabetes mellitus and the metabolic syndrome have a proatherogenic profile, which is indicated by lower levels of adiponectin and increased concentrations of A-FABP, as compared to healthy controls. In addition (and associated with the altered concentrations of the above adipocytokines) both patient groups have already entered into cardiac disease, which is indicated by increased troponin T levels. In contrast thereto, HIV infected patients on HAART have, compared to healthy controls, decreased adiponectin concentrations, but normal A-FABP levels and troponin T levels below the level of detectability, meaning there is no evidence of cardiac disorder. These findings are surprising as a substantial number of patients take drugs that may trigger the development of lipodystrophy, an important component of which is abdominal obesity.

Therefore, the above patient group was further analysed, see example 2.

**Example 2 Peptide marker levels in HIV infected subjects**

The subjects in above-identified group of HIV infected patients of example 1 received an antiretroviral treatment with various pharmaceuticals in various combinations. The pharmaceuticals are listed below.

The Table 2 hereinafter shows the A-FABP levels (median and ranges) of the individuals classified in tertiles (100 subjects each). The data are furthermore split up according to the classes of the administered antiretroviral pharmaceuticals.

Table 2:

|  | **A-FABP** [ng/ml] | | |
|---|---|---|---|
|  | 1. Terz. | 2. Terz. | 3. Terz. |
| **median** | 8.49 | 12.12 | 18.72 |

(continued)

| | A-FABP [ng/ml] | | |
|---|---|---|---|
| | 1. Terz. | 2. Terz. | 3. Terz. |
| range | 4.39-10.69 | 10.73-14.14 | 14.38-74.77 |
| *Category of medication/HAART* | N | | |
| I | 8 | 4 | 0 |
| I+II | 0 | 0 | 0 |
| I +III | 13 | 31 | 36 |
| I+II+III | 1 | 0 | 2 |
| I+II+IV | 1 | 0 | 0 |
| I+III+IV | 12 | 0 | 10 |
| II | 1 | 10 | 0 |
| II+III | 0 | 18 | 13 |
| II+III+IV | 2 | 4 | 0 |
| II+IV | 0 | 0 | 0 |
| III | 13 | 5 | 6 |
| III+IV | 26 | 5 | 12 |
| IV | 6 | 1 | 12 |
| I+IV | 0 | 0 | 0 |
| BMI | 21.92 | 22.96 | 24.17 |
| CRP [ng/ml] | 1.55 | 1.90 | 2.10 |
| troponin T median [pg/ml] | 0.0 | 0.0 | 0.0 |
| troponin T mean [pg/ml] | 1.0 | 1.4 | 4.9 |
| troponin T positive | 6/100 | 12/100 | 35/100 |
| Adiponectin | 2.1 | 1.8 | 1.5 |
| * 0.0 refers to values below cut-off (1.0 pg/ml) | | | |

BMI (body-mass-index)

category I: protease inhibitors (PIs); category II: non-nucleoside reverse transcriptase inhibitors (NNRTIs); category III: nucleoside reverse transcriptase inhibitors (NRTIs); category IV: nucleotide reverse transcriptase inhibitors (nRTIs)

Table 2 provides the following important information. When HIV infected persons are divided into A-FABP tertiles, it shows that high A-FABP concentrations are clearly associated with increased presence of detectable troponin T levels. This indicates that A-FABP is associated with the presence (and/or the risk) of a cardiac disorder also in HIV infected persons, although a subset of the individuals may have not yet developed increased levels of troponin T despite having already increased A-FABP levels.

Central obesity is a common feature of the metabolic syndrome and lipodystrophy ( the latter induced by certain HIV antiviral drugs). Therefore, both phenomena can often not be distinguished, and accordingly it may be challenging to tell if a given individual suffers from the one or the other disease. Furthermore, lipodystrophy is often hard to diagnose in its early stages. Occurrence of metabolic syndrome is associated with low adiponectin levels and increased A-FABP concentrations both being associated with a proatherogenic state such as increased troponin concentrations reflecting cardiac necrosis. Causes of metabolic alterations are difficult to diagnose in HIV infected patients as they may already suffer from metabolic syndrome and/or develop drug induced lipodystrophy (associated with abdominal obesity and peripheral wasting). BMI does not lend itself to be used as a symptom of the metabolic syndrome or of lipodystophy in these patients. In addition lipodystrophy in preferably induced by two classes or drugs: protease inhibitors and nucleoside reverse transcriptase analogues. As is evident from Table 2 increased and decreased A-FABP were associated with certain drugs or combination of drugs.

The following drug combinations were associated with increased A FABP levels: nRTIs; PIs + NRTIs; NNRTIs + NRTIs; PIs + NRTIs + nRTIs; (IV; I + III; II + III; I + III + IV)

The following drug combinations were associated with decreased ( or normal ) A FABP levels: PIs, NNRTIs + nRTIs ; NNRTIs + NRTIs + nRTIs; NRTIs ; NRTIs + nRTIs (I; II + III + IV ; III; III + IV)

A surprising finding of the study was that preferably drug combinations with nucleoside reverse transcriptase inhibitors but not with protease inhibitors (although both drugs induce lipodystrophy) were associated with increased A FABP levels. This indicates that clinical parameters (such as BMI or signs of lipodystrophy) fail to identify HIV infected individuals of increased risk for development of a cardiac disease.

### Example 3

A 48 year old male, who is HIV infected for at least 9.5 years is currently treated with a combination of NNRTIs and NRTIs for the past 3 years. He has no diabetes mellitus, coronary artery disease or impaired kidney function. The HIV-RNA is undetectable by current sensitive test and the CD4 count is 520 cell/microliter. A-FABP amount is increased (21 ng/ml), sensitive Troponin T amount is 5.5 pg/ml indicating the presence of a cardiac disease. According to his resistence profile Tenofovir is added to this combination (according to the results in Table 2) which may be a more favourable combination. 6 months thereafter his A-FABP has dropped to 14.5 ng/ml indicating the modification of the drug combination was effective in reducing the atherogenic profile, troponin T is measured with 5.2 pg/ml, HIV-RNA remained undetectable and CD 4 counts are unchanged indicating that the antiviral therapy was continuously effective. He is recommended to stay on this drug combination and to monitor the disease state in 6 more months

### Example 4

A 51 year old male who is infected with the HIV for the past 12 years, is currently on a combination of NNRTIs and NRTIs for 6 months, HIV RNA is undetectable, CD counts are 460 cells/microliter, no other comorbidities are known and specifically his kidney function is within normal. A-FABP is elevated (24 ng/ml) and troponin T is below the cut off value of the test, indicating that there was no evidence of a cardiac disease. Because of the proatherogenic profile but without evidence of cardiac disease he is switched to NRTIs and nRTIs according to resistance profile, 6 months later A F ABP has fallen to 17 ng/ml with troponin T still below cut off, HIV-RNA and CD 4 count have remained unchanged. He is recommended to stay on the drug combination and to return for follow up in 6 months.

### Example 5

A 54 year old male who is HIV positive for 13 years and without other comorbidities in on a combination of NRTIs and nRTIs for 4 years, HIV-RNA is undetectable and CD 4 counts are 560 cells/microliter. A-FABP amount is 11ng/ml, troponin T is undetectable. The patient is on an effective antiviral therapy as indicated by undetectable HIV-RNA levels and CD 4 count above 500 cell/microliter. In addition he does not exhibit a proatherogenic profile and thus he can be continued on the current medication. He is recommended to continue as is and to return for follow up in 6 to 12 months.

Summarizing the above, treatment of HIV infection has reached a significant level of complexity, the primary goal of treatment is the sustained suppression of HIV replication which results in maintenance of immunocompetence. Despite the development of HIV resistance, current antiviral drugs are able to achieve this in the majority of patients, thereby prolongating life. This extends the therapeutic focus to concomitant or drug induced diseases. Patients infected with the immunodeficiency virus require a lifelong therapy and increasingly reach an age where common cardiac risk factors become important (which are associated with an increased risk of cardiac complications such as myocardial infarction or heart failure). The present invention has identified A FABP as a drug associated marker associated with an increased levels of troponin T, the latter being associated with increased cardiac complications. Thus, the method of the present invention allows the selection of appropriate antiviral drug combinations in individuals at increased pre-existing risk and in case a drug combination is to be applied which increased A FABP and troponin identifies the need for monitoring. Thanks to the present invention cardiovascular risk assessment can be more effectively applied also in HIV patients where common and drug induced risk factors overlap and classical risk factors such as the metabolic syndrome are more difficult to identify.

Moreover if these drug combinations cannot be avoided duration of such treatment should be limited in time as assumingly drug induced heart disease represents a slow process.

**Claims**

1.  A method of diagnosing metabolic alterations in a HIV infected subject potentially leading to a cardiac complication or death, comprising the following steps:

    a) determining the amount of A-FABP or a variant thereof in a sample of the subject
    b) comparing the amount to a reference amount, and wherein based on the comparison carried out in step b) the metabolic alterations are diagnosed.

2.  The method according to claim 1, wherein the amount of adiponectin or a variant thereof is determined, further to the amount of A-FABP.

3.  The method according to claim 1 or 2, wherein an increased amount of A-FABP and/or a decreased amount of adiponectin is indicative of a metabolic alteration, with respect to reference amounts.

4.  The method according to any of claims 1 to 3, wherein the amount of CRP or a variant thereof is determined, further to the amount of A-FABP or a variant thereof and, optionally, adiponectin or a variant thereof.

5.  The method according to any of claims 1 to 4, wherein the amount of a cardiac troponin or a variant thereof is determined, further to the amount of A-FABP or a variant thereof and, optionally, adiponectin or a variant thereof and/or CRP or a variant thereof.

6.  The method according to any of claims 1 to 5, wherein the subject is a HIV-infected subject to whom an antiretroviral therapy is administered, preferably HAART.

7.  The method according to any of claims 1 to 6, wherein the metabolic alteration is a change in the lipid and/or glucose metabolism of the respective subject.

8.  A method of predicting the risk of a HIV infected subject to suffer from a cardiac complication or death, comprising the following steps:

    a) determining the amount of A-FABP or a variant thereof in a sample of the subject
    b) comparing the amount to reference amount, wherein based on the comparison carried out in step b) the risk is predicted..

9.  A method of monitoring the treatment of a HIV infected subject, comprising the steps of

    a) determining the amount of A-FABP or a variant thereof in a sample of the subject after therapy initiation at least once, or repeatedly within a given time interval;
    b) comparing the repeatedly determined amounts to at least one reference amount; wherein based on the comparison carried out in step b) the treatment is monitored.

10. A method of recommending or deciding on the adaptation of the treatment of a HIV infected subject, comprising the steps of

    a) determining the amount of A-FABP or a variant thereof in a sample of the subject after therapy initiation at least once, or repeatedly within a given time interval;
    b) comparing the repeatedly determined amounts to at least one reference amount;
    c) recommending or deciding on the treatment, based on the comparison carried out in step b).

11. The use of an antibody to A-FABP or a variant thereof for diagnosing metabolic alterations potentially leading to a cardiac complication or death.

12. A device for diagnosing metabolic alterations potentially leading to a cardiac complication or death and/or predicting the risk to suffer from a cardiac complication or death, in a HIV infected subject, comprising:

    a) an analyzing unit for determining the amounts of A-FABP or a variant thereof, and, as the case may be, adiponectin or a variant thereof and/or RBP-4 or a variant thereof and/or CRP or a variant thereof, in a sample of a subject;
b) an evaluation unit for comparing the amounts determined in step a) with reference amounts.

13. A kit for diagnosing metabolic alterations potentially leading to a cardiac complication or death and/or predicting the risk to suffer from a cardiac complication or death, in a HIV infected subject, comprising:

a) a specific ligand for determining the amounts of A-FABP or a variant thereof, and, as the case may be, adiponectin or a variant thereof and/or RBP-4 or a variant thereof and/or CRP or a variant thereof, in a sample of a subject;
b) an evaluation unit for comparing the amounts determined in step a) with reference amounts

14. The kit according to claim 13, additionally comprising a user's manual for interpreting the results of any measurement (s) with respect to diagnosing metabolic alterations potentially leading to a cardiac complication or death and/or predicting the risk to suffer from a cardiac complication or death

15. A method of assessing the metabolic effect and/or the potential to cause a cardiac complication, of an antiretroviral drug, comprising the steps of

a) determining the amount of A-FABP or a variant thereof in a sample of the subject
b) comparing the amount to reference amount
c) diagnosing metabolic alterations in the subject, based on the comparison carried out in step b);
d) assessing the metabolic effect and/or the potential to cause a cardiac complication of the antiretroviral drug.

Europäisches
Patentamt

European
Patent Office

Office européen
des brevets

## EUROPEAN SEARCH REPORT

Application Number

EP 11 15 7846

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | COLL B. ET AL.: "The fatty acid binding protein-4 (FABP4) is a strong biomarker of metabolic syndrome and lipodystrophy in HIV-infected patients", ATHEROSCLEROSIS, vol. 199, no. 1, July 2008 (2008-07), pages 147-153, XP022762416, [retrieved on 2007-11-05] | 1,6-15 | INV. G01N33/92 |
| Y | * abstract * * page 148, right-hand column, paragraph 1 * * paragraphs [0001], [03.2], [03.3], [0004] * * table 4 * | 2-7 | |
| X | ESCOTÉ X. ET AL.: "A study of fatty acid binding protein 4 in HIV-1 infection and in combination antiretroviral therapy-related metabolic disturbances and lipodystrophy", HIV MED., vol. 12, no. 7, August 2011 (2011-08), pages 428-437, XP002651817, [retrieved on 2011-01-19] | 1,6,7, 9-15 | |
| Y | * published online 19 January 2011 * * abstract * * page 429, right-hand column * * page 432, left-hand column, paragraph 2 - right-hand column, last paragraph * * page 434, left-hand column - right-hand column, last paragraph * * page 435, left-hand column, paragraph 2 * | 2-7 | TECHNICAL FIELDS SEARCHED (IPC) G01N |

-/--

The present search report has been drawn up for all claims

| Place of search | Date of completion of the search | Examiner |
|---|---|---|
| Munich | 20 July 2011 | Giry, Murielle |

EPO FORM 1503 03.82 (P04C01)

Europäisches
Patentamt

European
Patent Office

Office européen
des brevets

## EUROPEAN SEARCH REPORT

Application Number

EP 11 15 7846

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SAMARAS K. ET AL.: "Prevalence of metabolic syndrome in HIV-infected patients receiving highly active antiretroviral therapy using International Diabetes Foundation and Adult Treatment Panel III criteria", DIABETES CARE, vol. 30, no. 1, January 2007 (2007-01), pages 113-119, XP002651818, * abstract * * tables 1,3 * * page 117, middle column, last paragraph * * page 118, left-hand column, paragraph 1 * * page 118, middle column * ----- | 2-7 | |
| Y | SIERVO M. ET AL.: "Angiogenesis and biomarkers of cardiovascular risk in adults with metabolic syndrome", J. INTERN. MED., vol. 268, no. 4, October 2010 (2010-10), pages 338-347, XP002651819, * abstract * * page 343, left-hand column - right-hand column * * page 345, left-hand column, last paragraph - right-hand column, paragraph 1 * * page 346, left-hand column, last paragraph - right-hand column, paragraph 1 * ----- | 5-7 | |
| X Y | US 2009/181886 A1 (RUZICKA V. [CZ]) 16 July 2009 (2009-07-16) * abstract * * paragraphs [0001] - [0007], [0011], [0015] - [0018] * * claims 1-17 * ----- | 1,6-15 2-7 | TECHNICAL FIELDS SEARCHED (IPC) |

The present search report has been drawn up for all claims

| Place of search | Date of completion of the search | Examiner |
|---|---|---|
| Munich | 20 July 2011 | Giry, Murielle |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 11 15 7846

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2011

| Patent document cited in search report | Publication date | Patent family member(s) | Publication date |
|---|---|---|---|
| US 2009181886 A1 | 16-07-2009 | AT       468128 T<br>AU    2006321321 A1<br>CA       2614340 A1<br>EP       1904082 A2<br>WO    2007063363 A2<br>JP    2009501926 A | 15-06-2010<br>07-06-2007<br>07-06-2007<br>02-04-2008<br>07-06-2007<br>22-01-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008084003 A
- US 20070042424 A1
- US 5744305 A

**Non-patent literature cited in the description**

- **XU et al.** *Advances in Pharmacology,* 2010, vol. 60, 229-255
- **SWEENEY L.L. et al.** *AIDS,* 2007, vol. 21, 895-904
- **RANDELL et al.** *Antiviral Therapy,* 2010, vol. 16, 227-233
- **SWEENEY L.L. et al.** *AIDS,* 2007, 895-904
- **HAIDER et al.** *Clinical Pharmacology & Therapeutics,* vol. 81 (4), 580-585
- **FRIIS-MOLLER et al.** *N Engl J Med,* 2007, vol. 356, 1723-1735
- **STERNE JA et al.** *J Intern Med,* 2007, vol. 261, 255-267
- **ZOLOPA A.R.** *Antiviral Research,* 2010, vol. 85, 241-244
- **DOWDY ; WEARDEN.** Statistics for Research. John Wiley & Sons, 1983
- **OMLAND.** *Journal of Internal Medicine,* 2010, vol. 268, 207-217
- *J. Am. Coll. Cardiol.,* 2001, vol. 38, 2101-2113
- *J. Am. Coll. Cardiol.,* 2005, vol. 46, e1-e82
- **BAXA et al.** *Biochemistry,* 31 October 1989, vol. 28 (22), 8683-90
- **MAKOWSKI ; HOTAMISLIGIL.** *The Journal of Nutrition,* 2004, vol. 134, 2464S-2468S
- **HSU X. et al.** *Circulation Journal,* 2010, 327-331
- **XU A. et al.** *Circulation,* 2007, vol. 115, 1537-1543
- **TSO A A et al.** *Diabetes Care,* 2007, vol. 30, 2557-2672
- **RHEE RE. et al.** *Europ J Endocrinology,* 2009, vol. 160, 165-172
- **XU A. et al.** *Advances in Pharmacology,* 2010, vol. 60, 229-255
- **FURUHASHI M et al.** *Nature Reviews,* 2008, vol. 7, 489-503

- **HOTAMISLIGIL et al.** *Science,* 22 November 1996, vol. 274 (5291), 1377-1379
- **HAN et al.** *Journal of the American College of Cardiology,* vol. 49 (5), 531-8
- **KADOWAKI et al.** Adiponectin and adiponectin receptors in insulin resistance, diabetes, and the metabolic syndrome. *J Clin Invest.,* 2006, vol. 116 (7), 1784-1792
- **REXFORD S. AHIMA.** *Obesity,* 2006, vol. 14, 242S-249S
- **SMITH ; WATERMAN.** *Add. APL. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci.,* 1988, vol. 85, 2444
- **WOO.** *J. Biol. Chem.,* 1985, vol. 260 (24), 13384-13388
- **YCH.** *Circulation,* 2004, vol. 109, 11-1111-14
- **PEARSON ; LIPMAN.** *Proc. Natl. Acad Sci.,* 1988, vol. 85, 2444
- **ANDERSON.** *Circulation Research,* 1995, vol. 76 (4), 681-686
- **FERRIERES.** *Clinical Chemistry,* 1998, vol. 44, 487-493
- **MOSES et al.** *PNAS USA,* 1999, vol. 96 (6), 2645-2650
- **MASSON et al.** *Curr Heart Fail Rep,* 2010, vol. 7, 15-21
- **NOLAN.** *Trends Biotechnol.,* 2002, vol. 20 (1), 9-12
- **HANLEY et al.** *Radiology,* 1982, vol. 143, 29-36
- Clinical Practice Guidelines for chronic kidney disease. Am J. Kidney Dis. National Kidney Foundation, 2002, vol. 39